# EUROPÄISCHE PATENTSCHRIFT

(11) **EP 0 705 244 B1**
(45) Veröffentlichungstag und Bekanntmachung des Hinweises auf die Patenterteilung: **04.11.1998**
(21) Anmeldenummer: 94918734.8
(22) Anmeldetag: 24.06.1994
(51) Int. Cl.: C07C 323/59, C07C 323/60, C07D 295/18, C07K 5/02, A61K 31/16, A61K 31/535, A61K 38/00

(54) **NITRATO-AMINOSÄURE-DISULFIDE ZUR THERAPIE VON ERKRANKUNGEN DES HERZ-KREISLAUF-SYSTEMS**
NITRATO AMINO ACID DISULPHIDES FOR USE IN THE THERAPY OF DISORDERS OF THE CARDIOVASCULARY SYSTEM
DISULFURES D'ACIDES NITROAMINES POUR LE TRAITEMENT DE MALADIES DU SYSTEME CARDIO-VASCULAIRE

(30) Priorität: 26.06.1993 DE 4321306
(43) Veröffentlichungstag der Anmeldung: 10.04.1996
(73) Patentinhaber: SCHWARZ PHARMA AG, D-40789 Monheim/Rhld. (DE)
(72) Erfinder: SANDROCK, Klaus, D-40764 Langenfeld (DE); FEELISCH, Martin, D-40699 Erkrath (DE); BÖKENS, Hilmar, D-40593 Düsseldorf (DE); LEHMANN, Jochen, D-53115 Bonn (DE); MEESE, Claus, D-40789 Monheim (DE)
(86) Internationale Anmeldenummer: DE9400726
(87) Internationale Veröffentlichungsnummer: WO9500477

(56) Entgegenhaltungen:
- EP-A- 0 362 575
- EP-A- 0 451 760
- JOURNAL OF CARDIOVASCULAR PHARMACOLOGY, Bd. 22, Nr. 1, 1993, NEW YORK, US, Seiten 103 - 111 G. KOJDA, ET AL.: 'Nitric oxide liberating, soluble guanylate cyclase stimulating and vasorelaxing properties of the new nitrate-compound SPM 3672'

## Beschreibung

Die Erfindung betrifft neue, eine Disulfidgruppe enthaltende Nitrate und Verfahren zu deren Herstellung.

Organische Nitrate (Salpetersäureester) sind bewährte Arzneistoffe zur Behandlung von Herzerkrankungen. Sie entfalten ihre Wirkung sowohl durch eine Herzentlastung über eine Senkung von Vor- und Nachlast als auch eine Verbesserung der Sauerstoffversorgung für das Herz über eine Erweiterung der Koronarien.

In den vergangenen Jahren wurde jedoch festgestellt, daß die in der Therapie eingesetzten klassischen organischen Nitrate wie Glyceroltrinitrat, Isosorbiddinitrat oder Isosorbid-5-mononitrat bei kontinuierlicher Zufuhr hoher Dosen innerhalb kurzer Zeit eine deutliche Abschwächung der Wirkung, die Nitrattoleranz, aufweisen. Dieser Mangel konnte mit den durch EP 0 362 575 und EP 0 451 760 zur Verfügung gestellten Verbindungen behoben werden.

Entsprechend EP 0 362 575 und EP 0 451 760 wird die Wirkung der Nitroverbindungen über hieraus gebildete NO-Radikale vermittelt. Durch Reaktion der Nitrate mit der Thiolgruppe von Cystein kommt es dabei zunächst zur Ausbildung eines reaktiven und kurzlebigen, bisher noch hypothetischen Intermediärproduktes, das vermutlich der Thiolester der Salpetersäure oder ein Thionitrat ist, das intramolekular umlagert und nachfolgend in ein pharmakologisch aktives NO-Radikal und ein instabiles Thioradikal zerfällt, welches mit weiteren Thioradikalen zu dem entsprechenden Disulfid umgesetzt wird. Die Bildung und Freisetzung des NO-Radikals benötigt eine reduzierte Thiolgruppe enthaltendes Cystein, das zu Disulfiden umgesetzt wird, so daß die Abnahme der Wirkung von Nitroverbindungen bei andauernder Zufuhr oder Gabe hoher Dosen auf eine Erschöpfung des SH-Gruppenpools zurückgeführt wird. In EP 0 362 575 und EP 0 451 760 werden Verbindungen beansprucht, die Sulfhydrylgruppen enthalten und aufgrund dieses allgemeinen Bauprinzips eine Nitrattoleranz verhindern oder eine bereits eingetretene Nitrattoleranz abschwächen.

Überraschenderweise wurde nun gefunden, daß auch Nitroverbindungen, die anstelle einer freien Thiolgruppe eine Disulfidgruppe enthalten, wirksam sind und bei andauernder Zufuhr ebenfalls keine Nitrattoleranz aufweisen. Gegenstand der vorliegenden Erfindung sind daher Verbindungen der allgemeinen Formel I 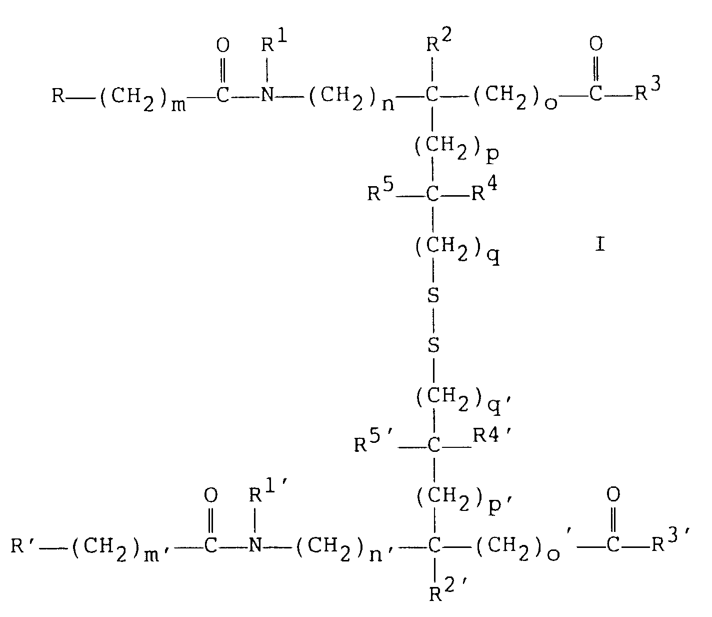 in der R und R' Gruppen der allgemeinen Formeln 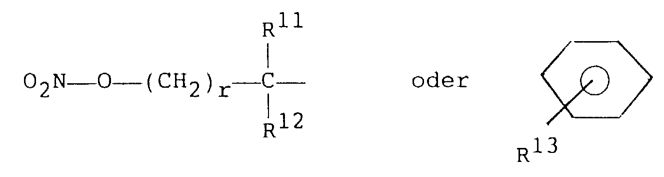 bedeuten, worin
- R¹¹: Wasserstoff, Alkyl mit 1 bis 6 Kohlenstoffatomen, substituiertes C₁-C₆-Niedrigalkyl, worin der Substituent Halogen, Hydroxy, C₁-C₆-Niedrigalkoxy, Aryloxy, Amino, C₁-C₆-Niedrigalkylamino, Acylamino, Acyloxy, Arylamino, Mercapto, C₁-C₆-Niedrigalkylthio, Arylthio ist
- R¹² wie R¹¹: Wasserstoff oder C₁-C₆-Niedrigalkyl bedeuten
- R¹³: Nitratoalkyl mit 1 bis 6 Kohlenstoffatomen ist
- r: die Zahlenwerte 0 bis 10 besitzt
und in der
- R¹, R^{1'}: Wasserstoff oder C₁-C₆-Niedrigalkyl bedeuten
- R², R^{2'}: Wasserstoff, C₁-C₆-Niedrigalkyl, Phenyl, Methoxyphenyl, Phenyl-C₁-C₆-niedrigalkyl, Methoxy-phenyl-C₁-C₆-niedrigalkyl, Hydroxyphenyl-C₁-C₆-niedrigalkyl, Hydroxy-C₁-C₆-niedrigalkyl, Alkoxy-C₁-C₆-niedrigalkyl, Amino-C₁-C₆-niedrigalkyl, Acylamino-C₁-C₆-niedrigalkyl, Mercapto-C₁-C₆-niedrigalkyl oder C₁-C₆-Niedrig-alkylthio-C₁-C₆-niedrigalkyl ist
- R³, R^{3'}: Hydroxy, C₁-C₆-Niedrigalkoxy, C₁-C₆-Niedrigalkenoxy, Di-C₁-C₆-niedrigalkylamino-C₁-C₆-niedrigalkoxy, Acylamino-C₁-C₆-niedrigalkoxy, Acyloxy-C₁-C₆-niedrigalkoxy, Aryloxy, Aryl-C₁-C₆-niedrigalkoxy, substituiertes Aryloxy oder substituiertes Aryl-C₁-C₆-niedrigalkoxy, worin der Substituent Methyl, Halogen oder Methoxy ist; Amino, C₁-C₆-Niedrigalkylamino, Di-C₁-C₆-niedrigalkylamino, Aryl-C₁-C₆-niedrig-alkylamino, Hydroxy-C₁-C₆-niedrigalkylamino, Pyrrolidin, Piperidin, Morpholin, Piperazin oder Aminosäurereste über Peptidverknüpfung sind
- R⁴, R^{4'}: Wasserstoff oder C₁-C₆-Niedrigalkyl ist,
- R⁵, R^{5'}: wie R⁴, R^{4'} Wasserstoff oder C₁-C₆-Niedrigalkyl bedeuten,
- R² und R³: sowie R^{2'} und R^{3'} miteinander unter Bildung eines Esters oder Amids verbunden sein können,
- R¹ und R²: sowie R^{1'} und R^{2'} miteinander unter Bildung einer Alkylenbrücke mit 2 bis 4 Kohlenstoffatomen, einer Alkylenbrücke mit 2 bis 3 Kohlenstoffatomen und einem Schwefelatom, einer Alkylenbrücke mit 3 bis 4 Kohlenstoffatomen, die eine Doppelbindung oder eine Alkylenbrücke wie oben erhält, substituiert durch Hydroxy, C₁-C₆-Niedrigalkoxy, C₁-C₆-Niedrigalkyl oder Di-C₁-C₆-niedrigalkyl, verbunden sein können,
- m, n, o, p, q: sowie m', n', o', p' und q' die Zahlenwerte 0 bis 10 besitzen
sowie deren physiologisch verträgliche Salze.

Weiterhin sind Gegenstand der vorliegenden Erfindung Verbindungen, die die folgenden Bezeichnungen tragen:
- N'-3-Nitratopivaloyl-L-cysteinamid-glutathion-mixeddisulfid,
- N'-3-Nitratopivaloyl-L-cysteinethylester-glutathion-mixeddisulfid,
- N'-3-Nitratopivaloyl-L-cysteinethylester-N'-acetyl-L-cystein-mixed-disulfid,
- N-(3-Nitratopivaloyl)-L-cysteinethylester-D,L-penicillaminmixed-disulfid,
- 2-Acetylamino-3-[2-(2,2-dimethyl-3-nitrooxy-propionylamino)-2-ethoxycarbonyl-ethyldisulfanyl]-3-methylbuttersäure.

Es handelt sich hierbei um asymmetrische Disulfide, die allgemein schwefelhaltige Aminosäuren, insbesondere Glutathion oder Penicillamin, enthalten.

Die erfindungsgemäßen Verbindungen können auch in Form ihrer Säure-Additionssalze vorliegen.

Nach einer Weiterbildung der Erfindung besitzt/en der/die aliphatische/n Teil/e der Nitratoalkylarylalkansäure und Nitratoalkansäurebestandteile eine Kettenlänge von 2-6 Kohlenstoffatomen; sie können geradkettig, verzweigt, racemisch oder optisch isomer sein.

Bevorzugt enthalten die erfindungsgemäßen Nitratoalkan- und Nitratoalkylarylalkan-Carbonsäurederivate der allgemeinen Formel (I) Disulfide von schwefelhaltigen Aminosäuren, insbesondere Cystin, Homocystin oder Penicillamindisulfid.

Nach einer weiteren Ausbildung der Erfindung liegen die Aminosäurendisulfide in der stereochemischen L- oder DL-Form vor.

Nach einer besonders vorteilhaften Weiterbildung der Erfindung weisen die beanspruchten Verbindungen die folgenden chemischen Formeln auf:
N,N'-Di-(3-Nitratopivaloyl)-L-cystin
N,N'-Di-(3-Nitratopivaloyl)-D,L-homocystin
N,N'-Di-(3-Nitratopivaloyl)-L-cystin-diethylester
N,N'-Di-(3-Nitratopivaloyl)-D,L-homocystin-diethylester
N,N'-Di-(3-Nitratopivaloyl)-L-cystin-di-tert.-butylester
N,N'-Di-(4-Nitratomethylbenzoyl)-L-cystin-dimethylester
N,N'-Di-(3-Nitratomethylbenzoyl)-L-cystin-dimethylester
N,N'-Di-(4-Nitratomethylbenzoyl)-L-cystin-di-(N,N'-butylamid)
N,N'-Di-(3-Nitratomethylbenzoyl)-L-cystin-di-(N,N'-butylamid)
N,N'-Di-(4-Nitratomethylbenzoyl)-L-cystindiamid
N,N'-Di-(3-Nitratomethylbenzoyl)-L-cystindiamid
N,N'-Di-(3-Nitratopivaloyl)-L-penicillamin-disulfid-diamid
N,N'-Di-(3-Nitratopivaloyl)-L-cystin-diamid
N,N'-Di-(3-Nitratopivaloyl)-L-cystin-di-(N,N'-methylamid)
N,N'-Di-(3-Nitratopivaloyl)-L-cystin-di-(N,N'-butylamid)
N,N'-Di-(3-Nitratopivaloyl)-L-cystin-di-(N,N'-tert.-butylamid)
N,N'-Di-(3-Nitratopivaloyl)-L-cystin-di-morpholid
N,N'-Di-(3-Nitratopivaloyl)-L-cystin-diisopropylester

Entsprechend EP 0 362 575 können die erfindungsgemäßen Verbindungen der allgemeinen Formel I in an sich bekannter Weise hergestellt werden, indem die entsprechende Nitratocarbonsäure oder deren reaktionsfähige Derivate mit der Aminogruppe eines freien, veresterten oder amidierten Aminosäuredisulfides kondensiert werden.

Verbindungen der allgemeinen Formel I, in der R³ und R^{3'} zusammen mit der benachbarten Carbonylgruppe eine Säureamidfunktion ausbilden, weisen gegenüber den bisher bekannten Nitraten eine verzögert einsetzende und lang anhaltende Wirkung auf. Dies hat eine verminderte Aktivierung der durch die Gefäßerweiterung hervorgerufenen physiologischen Gegenregulationen zur Folge und erlaubt eine Verlängerung des Applikationsintervalls. Insbesondere die Abschwächung kompensatorischer Mechanismen ist vorteilhaft, da sie eine Reduktion der unter einer Nitrattherapie auftretenden Nebenwirkungen wie reflektorische Tachykardie erwarten läßt.

Besonders bevorzugt sind daher Verbindungen der allgemeinen Formel I in der R³ und R^{3'} eine substituierte oder unsubstituierte Aminogruppe bedeuten.

Alternativ können die betreffenden Nitratoalkyl- oder Nitratoalkylarylalkyl-Carbonsäuren oder deren reaktive Derivate zunächst mit der Aminogruppe einer eine Thiolgruppe enthaltenden Aminosäure zum entsprechenden Säureamid kondensiert werden, welches anschließend mit Hilfe eines Oxidationsmittels wie Kaliumjodat unter Dimerisierung zu dem entsprechenden Disulfid der allgemeinen Formel I oxidiert wird.

Die Herstellung dieser Verbindungen gemäß diesen Methoden führt jedoch zu uneinheitlichen Produktgemischen, in denen die gewünschten Verbindungen nur in geringer Ausbeute enthalten sind und erfordern eine aufwendige Aufreinigung.

Diese Nachteile können dadurch vermieden werden, indem zunächst die entsprechenden Nitratocarbonsäuren oder deren reaktionsfähige Derivate mit den Aminogruppen freier über Disulfidbrücken verknüpften Aminosäuren kondensiert werden, die erhaltenen Verbindungen an ihren Carbonsäurefunktionen in aktivierte Ester überführt werden und diese anschließend mit Ammoniak, einem primären oder einem sekundären Amin zu den gewünschten Verbindungen umgesetzt werden. Gegenstand der Erfindung ist daher auch ein Verfahren zur Herstellung von Verbindungen der allgemeinen Formel II 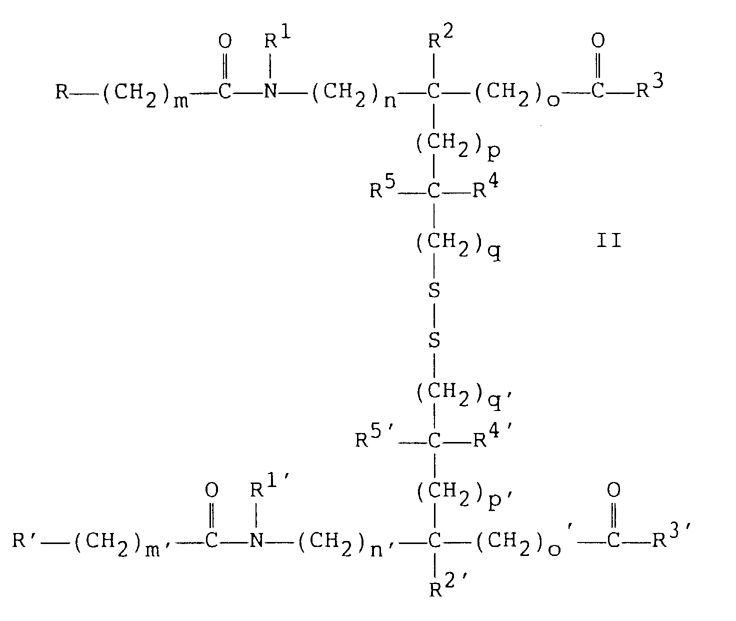 in der R und R' Gruppen der allgemeinen Formeln 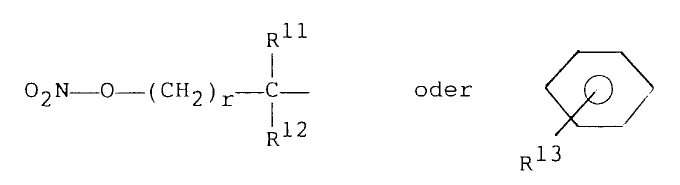 bedeuten,
worin
- R¹¹: Wasserstoff, Alkyl mit 1 bis 6 Kohlenstoffatomen, substituiertes C₁-C₆-Niedrigalkyl, worin der Substituent Halogen, Hydroxy, C₁-C₆-Niedrigalkoxy, Aryloxy, Amino, C₁-C₆-Niedrigalkylamino, Acylamino, Acyloxy, Arylamino, Mercapto, C₁-C₆-Niedrigalkylthio, Arylthio ist
- R¹² wie R¹¹: Wasserstoff oder C₁-C₆-Niedrigalkyl bedeuten
- R¹³: Nitratoalkyl mit 1 bis 6 Kohlenstoffatomen ist
- r: die Zahlenwerte 0 bis 10 besitzt
und in der
- R¹, R^{1'},: Wasserstoff oder C₁-C₆-Niedrigalkyl bedeuten
- R², R^{2'}: Wasserstoff, C₁-C₆-Niedrigalkyl, Phenyl, Methoxyphenyl, Phenyl-C₁-C₆-niedrigalkyl, Methoxy-phenyl-C₁-C₆-niedrigalkyl, Hydroxyphenyl-C₁-C₆-niedrigalkyl, Hydroxy-C₁-C₆-niedrigalkyl, Alkoxy-C₁-C₆-niedrigalkyl, Amino-C₁-C₆-niedrigalkyl, Acylamino-C₁-C₆-niedrigalkyl, Mercapto-C₁-C₆-niedrigalkyl oder C₁-C₆-Niedrigalkylthio-C₁-C₆-niedrigalkyl ist
- R³, R^{3'}: Amino, C₁-C₆-Niedrigalkylamino, Di-C₁-C₆-niedrigalkylamino, Aryl-C₁-C₆-niedrigalkylamino, Hydroxy-C₁-C₆-niedrigalkylamino, Pyrrolidin, Piperidin, Morpholin, Piperazin oder Aminosäurereste über Peptidverknüpfung sind
- R⁴, R^{4'}: Wasserstoff oder C₁-C₆-Niedrigalkyl ist,
- R⁵, R^{5'}: wie R⁴, R^{4'} Wasserstoff oder C₁-C₆-Niedrigalkyl bedeuten,
- R¹ und R²: sowie R^{1'} und R^{2'} miteinander unter Bildung einer Alkylenbrücke mit 2 bis 4 Kohlenstoffatomen, einer Alkylenbrücke mit 2 bis 3 Kohlenstoffatomen und einem Schwefelatom, einer Alkylenbrücke mit 3 bis 4 Kohlenstoffatomen, die eine Doppelbindung oder eine Alkylenbrücke wie oben enthält, substituiert durch Hydroxy, C₁-C₆-Niedrigalkoxy, C₁-C₆-Niedrigalkyl oder Di-C₁-C₆-niedrigalkyl, verbunden sein können,
- m, n, o, p, q: sowie m', n', o', p' und q' die Zahlenwerte 0 bis 10 besitzen,
das dadurch gekennzeichnet ist, daß Verbindungen der allgemeinen Formel III 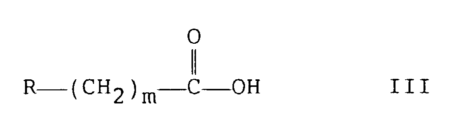 in der R eine Gruppe der allgemeinen Formel 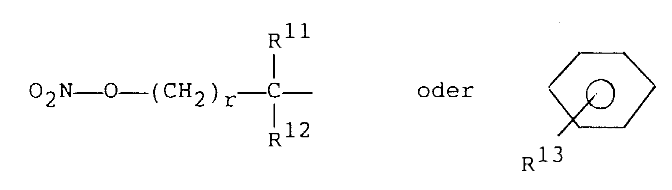 bedeutet, worin
- R¹¹: Wasserstoff, Alkyl mit 1 bis 6 Kohlenstoffatomen, substituiertes C₁-C₆-Niedrigalkyl, worin der Substituent Halogen, Hydroxy, C₁-C₆-Niedrigalkoxy, Aryloxy, Amino, C₁-C₆-Niedrigalkylamino, Acylamino, Acyloxy, Arylamino, Mercapto, C₁-C₆-Niedrigalkylthio, Arylthio ist
- R¹² wie R¹¹: Wasserstoff oder C₁-C₆-Niedrigalkyl bedeuten
- R¹³: Nitratoalkyl mit 1 bis 6 Kohlenstoffatomen ist
- m und r: die Zahlenwerte 0 - 10 besitzen
in Form ihrer freien Säuren, reaktiven Säurehalogenide, Säure-Azide, Ester und Säure-Anhydride in an sich bekannter Weise mit Verbindungen der allgemeinen Formel IV 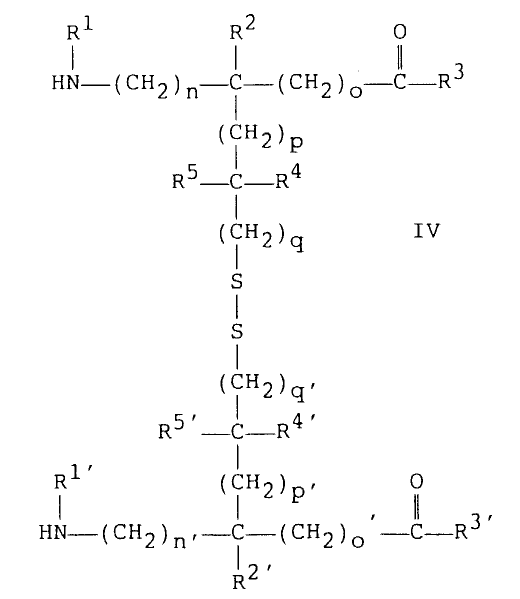 in denen
- R¹, R^{1'}: Wasserstoff oder C₁-C₆-Niedrigalkyl bedeuten
- R², R^{2'}: Wasserstoff, C₁-C₆-Niedrigalkyl, Phenyl, Methoxyphenyl, Phenyl-C₁-C₆-niedrigalkyl, Methoxy-phenyl-C₁-C₆-niedrigalkyl, Hydroxyphenyl-C₁-C₆-niedrigalkyl, Hydroxy-C₁-C₆-niedrigalkyl, Alkoxy-C₁-C₆-niedrigalkyl, Amino-C₁-C₆-niedrigalkyl, Acylamino-C₁-C₆-niedrigalkyl, Mercapto-C₁-C₆-niedrigalkyl oder C₁-C₆-Niedrig-alkylthio-C₁-C₆-niedrigalkyl ist
- R³, R^{3'}: Hydroxy oder Halogen bedeutet
- R⁴, R^{4'}: Wasserstoff oder C₁-C₆-Niedrigalkyl ist,
- R⁵, R^{5'}: wie R⁴, R^{4'} Wasserstoff oder C₁-C₆-Niedrigalkyl bedeuten,
- R¹ und R²: sowie R^{1'} und R^{2'} miteinander unter Bildung einer Alkylenbrücke mit 2 bis 4 Kohlenstoffatomen, einer Alkylenbrücke mit 2 bis 3 Kohlenstoffatomen und einem Schwefelatom, einer Alkylenbrücke mit 3 bis 4 Kohlenstoffatomen, die eine Doppelbindung oder eine Alkylenbrücke wie oben enthält, substituiert durch Hydroxy, C₁-C₆-Niedrigalkoxy, C₁-C₆-Niedrigalkyl oder Di-C₁-C₆-niedrigalkyl, verbunden sein können,
- n, o, p, q: sowie n', o', p' und q' die Zahlenwerte 0 bis 10 besitzen
zu Verbindungen der allgemeinen Formel V 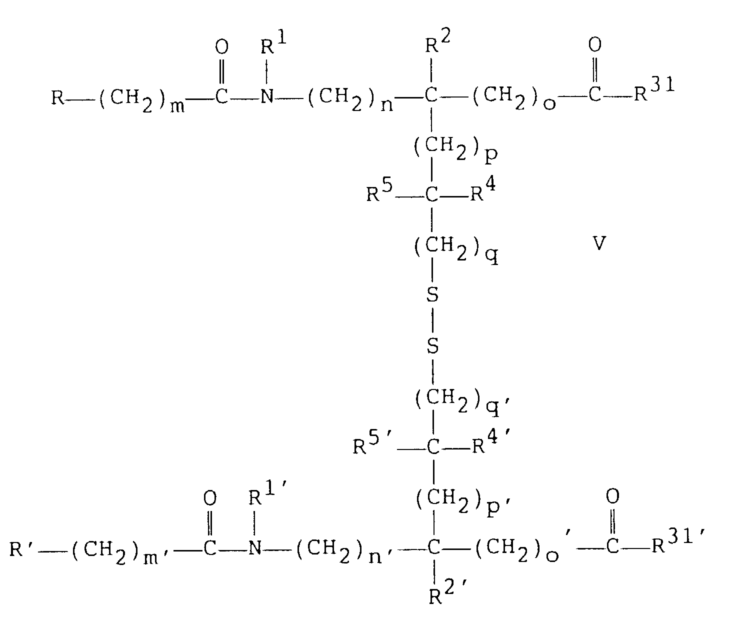 kondensiert werden, diese an ihren freien oder halogenierten Carbonsäurefunktionen mit N-Hydroxysuccinimid zu aktivierten Carbonsäure-N-hydroxysuccinimidester der allgemeinen Formel VI 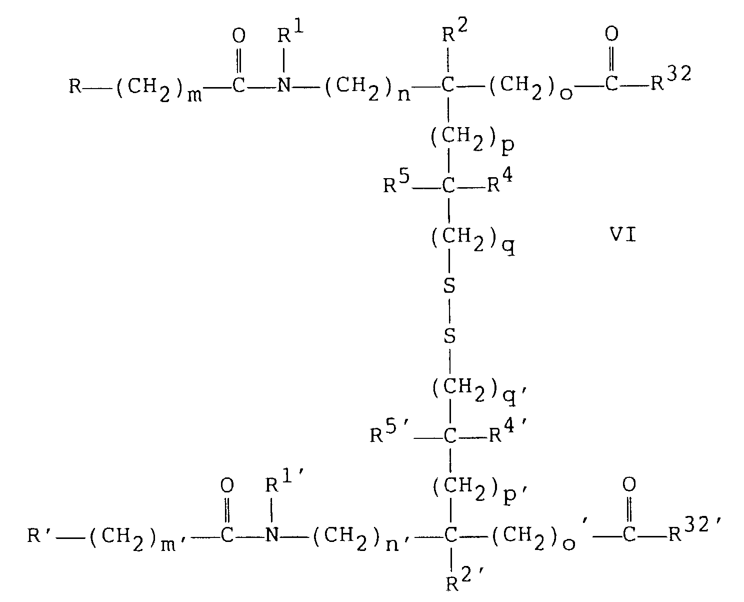 überführt werden,
in denen
- R³² und R^{32'}: abweichend von der oben genannten Bedeutung 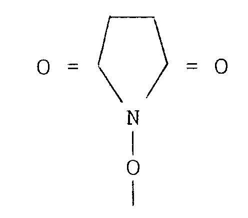
bedeutet
und diese anschließend mit Ammoniak, einem primären oder einem sekundären Amin, insbesondere mit C₁-C₆-Niedrigalkylamin, Di-C₁-C₆-niedrigalkylamin, Aryl-C₁-C₆-niedrigalkylamin, Hydroxy-C₁-C₆-niedrigalkylamin, Pyrrolidin, Piperidin, Morpholin, Piperazin oder Aminosäuren zu den Verbindungen der allgemeinen Formel II umgesetzt werden.

Die in den allgemeinen Formeln genannten "niederen Alkylreste" sind unverzweigte oder verzweigte Kohlenwasserstoffreste mit höchstens 6 Kohlenstoffatomen. Spezielle Beispiele sind die Methyl-, Ethyl-, Propyl-, Isopropyl-, Butyl, Isobutyl-, tert. Butyl-, Pentyl- und Isopentylgruppe. Die niederen Alkoxyreste enthalten ebenfalls höchstens 6 Kohlenstoffatome. Spezielle Beispiele sind die Methoxy-, Ethoxy-, Propoxy-, Isopropoxy-, Butoxy-, Isobutoxy- und tert.-Butoxygruppe. Bevorzugte niedere Alkyl- oder Alkoxyreste enthalten höchstens 4 Kohlenstoffatome. Besonders bevorzugt sind Reste mit 2 Kohlenstoffatomen. Der bevorzugte phenylsubstituierte niedrige Alkylrest ist die Phenylmethylgruppe oder die Phenylethylgruppe.

Die "niederen Alkanoylreste" sind Acylreste von Fettsäuren mit 2-6 Kohlenstoffatomen.

Als Halogenatome kommen Fluor-, Chlor-, Brom- und Jodatome in Frage. Bevorzugt sind Chlor- und Bromatome.

"Aryl-" bedeutet Benzyl- oder Phenylreste.

Die substituierten Benzyl- oder Phenylreste tragen den Substituenten vorzugsweise in 4-Stellung.

Die hydroxysubstituierten niederen Alkylreste besitzen eine Hydroxylgruppe an einem Alkylrest der vorstehend beschriebenen Art, vorzugsweise an endständigen Kohlenstoffatomen. Spezielle Beispiele sind die Hydroxymethyl- und die 2-Hydroxyethylgruppe.

Die erfindungsgemäßen neuen Verbindungen der allgemeinen Formel I und II sowie ihre Salze können in flüssiger oder fester Form oral, enteral oder parenteral appliziert werden.

Als Injektionsmedium kommt vorzugsweise Wasser zur Anwendung, welches die bei Injektionslösungen üblichen Zusätze wie Stabilisierungsmittel, Lösungsvermittler oder Puffer enthält. Derartige Zusätze sind z.B. Tartrat- und Citratpuffer, Ethanol, Komplexbildner (wie Ethylendiamintetraessigsäure und deren nicht toxische Salze), hochmolekulare Polymere (wie flüssiges Polyethylenoxid) zur Viskositätsregulierung. Feste Trägerstoffe sind z.B. Stärke, Lactose, Mannit, Methylcellulose, Talkum, hochdisperse Kieselgelsäuren, höhermolekulare Fettsäuren (wie Stearinsäure), Gelatine, Agar-Agar, Calciumphosphat, Magnesiumstearat, tierische und pflanzliche Fette und feste hochmolekulare Polymere (wie z.B. Polyethylenglykole); für orale Applikation geeignete Zubereitungen können gewünschtenfalls Geschmacks- und Süßstoffe enthalten.

Nach einer weiteren Ausbildung der Erfindung weisen Arzneimittel einen Gehalt an einer und/oder einem Gemisch der erfindungsgemäßen Verbindungen auf.

Diese Arzneimittel können zur Behandlung von Erkrankungen des Herz-Kreislaufsystems, beispielsweise als Mittel zur Behandlung der koronaren Herzkrankheit, des Blut-Hochdrucks und der Herzinsuffizienz sowie zur Erweiterung der peripheren Gefäße, einschließlich der Hirn- und Nierengefäße eingesetzt werden.

Pharmazeutische Zubereitungen, eine vorausberechnete Menge einer oder mehrerer der erfindungsgemäßen Verbindungen enthaltend, können einmal täglich in Form retardierter Zubereitungen oder mehrmals täglich in regelmäßigen Intervallen (2-3 mal täglich) verabreicht werden. Die überlicherweise pro Tag applizierten Wirkstoffmengen sind 20 - 300 mg pro Tag, bezogen auf 75 kg Körpergewicht. In Form von Injektionen können die erfindungsgemäßen Verbindungen 1-8 mal pro Tag bzw. durch Dauerinfusion gegeben werden. Normalerweise reichen Mengen von 5-200 mg/Tag aus.

Eine typische Tablette kann folgende Zusammensetzung aufweisen:

| | | |
|---|---|---|
| 1) | N,N'-Di-(3-Nitratopivaloyl)-L-cystindiamid | 30 mg |
| 2) | Stärke, U.S.P. | 57 mg |
| 3) | Lactose, U.S.P. | 73 mg |
| 4) | Talkum, U.S.P. | 9 mg |
| 5) | Stearinsäure | 6 mg |

Die Stoffe 1, 2 und 3 werden gesiebt, granuliert, mit 4 und 5 homogen gemischt und anschließend tablettiert.

Die Ausführungsbeispiele erläutern die Erfindung, ohne darauf beschränkt zu sein.

### Beispiel 1

### Darstellung von N,N'-Di-(3-Nitratopivaloyl)-L-cystin

149,1 g (3,727 Mol) Natriumhydroxid wurden in 2,5 1 Wasser gelöst, auf 0°C gekühlt und unter Rühren und Eisbadkühlung 448,0 g (1,864 Mol) L-Cystin portionsweise zugegeben, wobei 5°C nicht überschritten wurden. Hierzu wurden 2,0 1 Dichlormethan zugegeben. Anschließend wurden unter Kühlung und starkem Rühren gleichzeitig Lösungen, bestehend aus 734,0 g (3,961 Mol) 3-Nitratopivaloylchlorid in 1,0 1 Dichlormethan und 167,8 g (4,195 Mol) Natriumhydroxid in 1,0 1 Wasser langsam zugetropft, wobei die Temperatur auf maximal 29°C stieg. Nach zweieinhalbstündigem Rühren bei Raumtemperatur wurde die Dichlormethan-Phase abgetrennt, die Wasser-Phase mit 1,0 1 Dichlormethan gewaschen und unter Rühren mit 350 ml 37 %iger (4,227 Mol) Salzsäure versetzt, wobei ein öliges Produkt ausfiehl. Nach dreimaliger Extraktion dieses Gemisches mit je 2,0 1 Ethylacetat wurden die Extrakte vereinigt und am Rotavapor® eingeengt, wobei ein brauner, öliger Rückstand zurückblieb. Dieser wurde in 2,0 l siedendem Ethylacetat gelöst und bei stark vermindertem Druck (Ölpumpenvakuum) und einer Badtemperatur von ca. 55°C bis zur Gewichtskonstanz eingeengt (Produkt schäumt stark). Der dabei erhaltene Rückstand wurde in 1,0 1 siedendem Ethylacetat gelöst, auf Raumtemperatur abgekühlt, mit 1,0 1 Dichlormethan verdünnt und unter Zugabe von Impfkristallen im Gefrierschrank bei ca. -30°C zur Kristallisation gebracht. Die auskristallisierte Substanz wurde abgesaugt, zweimal mit je 250 ml einer Lösung von 300 ml Dichlormethan und 200 ml Ethylacetat von ca. -30°C gewaschen und im Vakuumtrockenschank bei ca. 40°C bis zur Gewichtskonstanz getrocknet.

Die Ausbeute betrugt 318,03 g (entsprechend 32,16 % d.Th.).
Fp: 47,2°C

### Beispiel 2

### Darstellung von N,N'-Di-(3-Nitratopivaloyl)-D,L-Homocystin

Die Darstellung und Aufarbeitung erfolgte analog Beispiel 1. Beim Ansäuern mit 37 %-iger Salzsäure fiel festes Produkt aus, das wie beschrieben mit Ethylacetat extrahiert und eingeengt wurde, wobei 51,3 g weißes, festes Produkt (entsprechend 73,5 % d.Th.) zurückblieben.

Dieses wurde unter Erwärmen in 400 ml Methanol gelöst, filtriert und auf Raumtemperatur abgekühlt. Hierzu wurden unter Rühren 400 ml dest. Wasser zugegeben und 3 Std. bei Raumtemperatur gerührt, wobei langsam ein Produkt auskristallisierte. Dieses wurde abgesaugt, einmal mit einem Gemisch, bestehend aus 60 ml dest. Wasser und 40 ml Methanol gewaschen und anschließend im Vakuumtrockenschrank bei ca. 40°C und ca. 2 Torr bis zur Gewichtskonstanz getrocknet.

Die Ausbeute betrug 31,3 g (entsprechend 44,8 % d.Th.).
Fp: ca. 42°C

### Beispiel 3

### Darstellung von N,N'-Di-(3-Nitratopivaloyl)-L-cystindiethylester

24,4 g (82,9 mmol) 3-Nitratopivaloyl-L-cysteinethylester wurden bei Raumtemperatur in 150 ml Eisessig gelöst und unter Rühren eine Lösung von 2,9 g (13,9 mmol) Kaliumjodat in 300 ml dest. Wasser langsam zugetropft, wobei sich der Ansatz bräunlich verfärbte und nach 15 Min. Rühren eine kristalline Substanz ausfiel. Die Substanz wurde abgesaugt, in 150 ml Dichlormethan gelöst und nacheinander je einmal mit 100 ml 1 %-iger Natriumthiosulfat-Lösung, 100 ml 1 n Salzsäure und 100 ml dest. Wasser gewaschen. Die Dichlormethan-Phase wurde eingeengt, der verbleibende Rückstand (25,4 g) in 150 ml Methanol gelöst und mit 45 ml dest. Wasser versetzt. Nach Zugabe von Impfkristallen kristallisierte die Substanz bei Raumtemperatur. Zur Vervollständigung der Kristallisation wurde der Kolben über Nacht in den Kühlschrank (ca. 0°C) gestellt. Die abgesaugte Substanz wurde zweimal mit je 30 ml eines ca. -25°C kalten Gemisches, bestehend aus Methanol und dest. Wasser im Verhältnis 1:1 gewaschen und im Vakuumtrockenschrank über Phosphorpentoxid bei ca. 35°C und ca. 250 Pa bis zur Gewichtskonstanz getrocknet.

Die Ausbeute betrug 16,9 g (entsprechend 69,5 % d.Th.).
Fp: 84,9°C

### Beispiel 4

### Darstellung von Di-(Nitratopivaloyl)-D,L-Homocystindiethylester

11,4 g (35 mmol) Homocystindiethylester und 12,0 g (73,5 mmol) 3-Nitratopivalinsäure wurden in 200 ml Dichlormethan gelöst, auf ca. 10°C gekühlt und unter Rühren eine Lösung von 15,2 g (73,5 mmol) DCC in 100 ml Dichlormethan in ca. 15 Min. zugetropft, wobei gegen Ende des Zutropfens DCC-Harnstoff ausfiel. Anschließend wurde über das Wochenende bei Raumtemperatur gerührt, der Harnstoff abgesaugt und zweimal mit je 50 ml Dichlormethan gewaschen. Die vereinigten Dichlormethan-Lösungen wurden zweimal mit 100 ml 1 n Salzsäure, zweimal mit je 100 ml Natriumhydrogencarbonat-Lösung und einmal mit 100 ml dest. Wasser gewaschen, im Rotavapor® eingeengt und der dabei erhaltene Rückstand unter Erwärmen in einem Gemisch, bestehend aus 175 ml Ethanol abs. und 25 ml Ethylacetat gelöst. Die erhaltene Lösung wurde filtriert, über Nacht in den Kühlschrank (0°C) gestellt. Die dabei gebildeten Kristalle wurden abgesaugt und nach zweimaligem Waschen mit je 20 ml Ethanol abs. von ca. 0°C im Vakuumtrockenschrank bei Raumtemperatur und ca. 250 Pa bis zur Gewichtskonstanz getrocknet.

Die Ausbeute betrug 12,4 g (entsprechend 57,6 % d.Th.).
Fp: 120,4°C

### Beispiel 5

### Darstellung von N,N'-Di-(3-Nitratopivaloyl)-L-cystin-di-tert.-butylester

17,76 g (0,03 Mol) L-Cystin-di-tert.-butylester und 10,36 g (0,126 Mol) Natriumacetat wurde in 80 ml Wasser und 120 ml Dichlormethan gelöst und bei 10°C bis 15°C eine Lösung von 12,0 g (0,066 Mol) 3-Nitratopivaloylchlorid und 50 ml Dichlormethan zugetropft. Dieser Ansatz wurde über Nacht bei Raumtemperatur gerührt, die organische Phase abgetrennt und diese mit jeweils 50 ml 1 n Salzsäure und konz. Natriumhydrogencarbonat-Lösung gewaschen. Nach Trocknen über Natriumsulfat wurde filtriert und einrotiert.

Der verbliebene Rückstand wurde aus Ethanol umkristallisiert, welcher über Nacht bei -25°C vervollständigt wurde und einmal mit 10 ml Ethanol (-25°C) gewaschen wurde.

Die Ausbeute betrug 12,73 g (entsprechend 66,02 % d.Th.).
Fp: 93,7°C

### Beispiel 6

### Darstellung von N,N'-Di-(4-Nitratomethylbenzoyl)-L-cystindimethylester

1,48 g (0,055 Mol) L-Cystindimethylester-Base wurden unter Rühren in 10 ml Methylenchlorid gelöst, 0,56 g (0,055 Mol) Triethylamin zugefügt, anschließend eine Lösung von 2,16 g (0,01 mol) 4-Nitratomethylbenzoylchlorid in 10 ml Methylenchlorid langsam zugetropft. Nach 2 h Rühren wurde das Produkt abgesaugt, mit 2 x 15 ml 1 n Salzsäure und 2 x 15 ml Wasser gewaschen, getrocknet und aus Ethylacetat umkristallisiert, wobei farblose Kristalle erhalten wurden.

Die Ausbeute betrug 1,89 g (entsprechend 60,4 % d.Th.).
Fp.: 155°C

### Beispiel 7

### Darstellung von N,N'-Di-(3-Nitratomethylbenzoyl)-L-cystindimethylester

Die Darstellung erfolgte analog dem 4-Nitratomethylbenzoylderivat. Da hier das Rohprodukt im Methylenchlorid des Reaktionsansatzes gelöst blieb, wurde nach 2 h Rühren mit 15 ml 1 n Salzsäure und 2 x 15 ml Wasser gewaschen, getrocknet, i.Vak. eingedampft und anschließend aus Diisopropylether umkristallisiert, wobei farblose Nadeln erhalten wurden.

Die Ausbeute betrug 2,68 g (entsprechend 85,6 % d.Th.).
Fp.: 91°C

### Beispiel 8

### Darstellung von Di-N,N'-(4-Nitratomethylbenzoyl)-L-cystin-di-N,N'-butylamid

1,23 g (0,0035 Mol) L-Cystin-di-N,N'-butylamid-Base wurden in 20 ml Methylenchlorid gelöst, 0,61 g (0,006 Mol) Triethylamin zugesetzt und eine Lösung von 1,29 g (0,006 Mol) 4-Nitratomethylbenzoylchlorid in 10 ml Methylenchlorid langsam zugetropft. Nach 2 h Rühren wurde mit 20 ml 1 n Salzsäure und 2 x 15 ml Wasser gewaschen, über Natriumsulfat getrocknet, i.Vak. zur Trockne eingeengt und aus Methylenchlorid umkristallisiert, wobei ein farbloses Pulver erhalten wurde.

Die Ausbeute betrug 0,93 g (entsprechend 26,3 % d.Th.).
Fp.: 183°C

### Beispiel 9

### Darstellung von (S,S)-Bis-N'N'-(3-Nitratomethylbenzoyl)-cystin-di-N N'-butylamid

erfolgte analog dem 4-Nitratomethylbenzoyl-Derivat.
Aussehen: Farbloses Pulver

Die Ausbeute betrug 0,4 g (entsprechend 11,3 % d.Th.).
Fp: 174-175°C

### Beispiel 10

### Darstellung von N,N'-Di-(4-Nitratomethylbenzoyl)-cystindiamid

1,03 g (0,0035 Mol) Cystindiamid-Base wurden in 20 ml Methylenchlorid supendiert, 0,60 g (0,006 mol) Triethylamin zugesetzt und eine Lösung von 1,29 g (0,006 mol) 4-Nitratomethylbenzoylchlorid in 10 ml Methylenchlorid langsam zugetropft. Nach Rühren über Nacht wurde mit 20 ml 1 n Salzsäure und 2 x 20 ml Wasser gewaschen, über Natriumsulfat getrocknet, einrotiert und der Rückstand aus Methylenchlorid umkristallisiert, wobei ein farbloses Pulver erhalten wurde.

Die Ausbeute betrug 0,32 g (entsprechend 15,3 % d.Th.).
Fp.: 143°C

### Beispiel 11

### Darstellung von (S,S)-N,N'-Bis-(3-Nitratomethylbenzoyl)-cystindiamid

erfolgte analog dem 4-Nitratomethylbenzoyl-Derivat, wobei ein farbloses Pulver erhalten wurde.

Die Ausbeute betrug 0,25 g (entsprechend 12,2 % d.Th.).
Fp: 136°C

### Beispiel 12

### Darstellung von N,N'-Di-(3-Nitratopivaloyl)-L-penicillamindisulfid-diamid

### 1. Stufe: Herstellung von L-Penicillamindisulfidbisamid

In ca. 100 ml flüssigen Ammoniak wurden bei -40 bis -55°C 7,95 g (0,02 Mol) L-Penicillamindisulfiddimethylester x 2 HCl eingetragen, über Nacht bei -40 bis -30°C gerührt und das Kühlbad entfernt, worauf der Ammoniak abdampfte. Anschließend wurde der Ansatz am Rotavapor® bei einer Badtemperatur von 50°C eingeengt, der gesamte Rückstand in 40 ml warmen Isopropanol aufgenommen und filtriert. In das Filtrat wurde HCl-Gas eingeleitet, wobei das Produkt ausfiel, welches abgesaugt wurde.

Die Ausbeute betrug 4,6 g (entsprechend 62,61 % d.Th.).

### 2. Stufe: Herstellung von N,N'-Di-(3-Nitratopivaloyl)-L-penicillamindisulfid-diamid

4,41 g (12 mmol) L-Penicillamindisulfidbisamid x 2 HCl und 4,03 g (48 mmol) Natriumhydrogencarbonat wurden in 100 ml Wasser gelöst, 60 ml Dichlormethan zugegeben und solange gerührt, bis beide Phasen klar gelöst waren. Dann wurde bei Raumtemperatur eine Lösung von 4,36 g (24 mmol) 3-Nitratopivaloylchlorid in 60 ml Dichlormethan zugetropft, über Nacht gerührt, die organische Phase abgetrennt und zweimal mit je 50 ml 1 n Salzsäure gewaschen. Das Dichlormethan wurde abgezogen, wobei ein öliger Rückstand zurückblieb, der säulenchromatographisch aufgereinigt wurde.

Die Ausbeute betrug 3,45 g (entsprechend 49,17 % d.Th.).

### Beispiel 13

### Darstellung von N,N'-Di-(3-Nitratopivaloyl)-L-cystindiamid

### 1. Stufe: Darstellung von N,N'-Di-(3-Nitratopivaloyl)-L-cystin-di-(N-hydroxysuccinimidester)

477,5 g (0,9 Mol) N,N'-Di-(3-Nitratopivaloyl)-L-cystin (Beispiel 1) und 213,4 g (1,854 Mol) N-Hydroxysuccinimid wurden in 1400 ml Tetrahydrofuran gelöst, auf 5°C gekühlt und unter Rühren und Eisbadkühlung eine Lösung von 382,5 g (1,854 Mol) DCC in 600 ml Tetrahydrofuran in 30 Min. zugetropft. Hierbei stieg die Temperatur des Ansatzes auf 30°C und kristallisierte, so daß er zur Aufrechterhaltung des Rührens zunächst mit 0,5 1 Tetrahydrofuran versetzt, dann auf ca. 50°C erwärmt und anschließend mit 0,5 1 Aceton nochmals verdünnt wurde. Hierauf wurde ca. 30 Min. bei ca. 50 °C gerührt, der DCC-Harnstoff abgesaugt (393,1 g getrocknet = 94,5 % d.Th.) und mit 0,5 1 Aceton gewaschen. Die vereinigten organischen Lösungen wurden am Rotavapor® bis zur Trockene eingeengt, der Rückstand in 1,7 l Ethylacetat unter Refluxieren gelöst und die dabei verbliebenen DCC-Harnstoff-Reste (7,5 g = 1,8 % d.Th.) abfiltriert. Nach Stehen über Nacht bei Raumtemperatur wurde die auskristallisierte Substanz abgetrennt, zweimal mit je 0,2 l Ethylacetat gewaschen und im Vakuumtrockenschrank bei Raumtemperatur und ca. 250 Pa bis zur Gewichtskonstanz getrocknet.

Die Ausbeute betrugt 373,9 g (entsprechend 57,3 % d.Th.)

### 2. Stufe: Darstellung von N,N'-Di-(3-Nitratopivaloyl)-L-cystindiamid

72,4 g (0,1 Mol) N,N'-Di-(3-Nitratopivaloyl)-L-cystin-di-(N-hydroxysuccinimidester) wurden in 300 ml Ethylacetat gelöst. Hierzu wurde unter Rühren eine Lösung, bestehend aus 16,5 ml (25 %iger) Ammoniaklösung und 60 ml Wasser, zugetropft, 15 Min. nachgerührt, nochmals eine Lösung, bestehend aus 1,65 ml (25 %iger) Ammoniaklösung und 6 ml Wasser, zugegeben und 30 Min. nachgerührt.

Nach Abtrennung der organischen Phase wurde mit 100 ml Wasser gewaschen, mit Natriumsulfat getrocknet, mit Aktivkohle geklärt und das Lösungsmittel vollständig abgezogen, wobei 56,72 g Rückstand verblieben. Dieser wurde in 90 ml Methanol gelöst, mit 40 ml Wasser versetzt und bei -25°C umkristallisiert.

Das ausgefallene Produkt wurde abgesaugt und zweimal mit je 25 ml Methanol/Wasser 1:1 gewaschen.

Die Ausbeute betrug 37,4 g (entsprechend 70,75 % d.Th.).
Fp: 82,3°C

### Beispiel 14

### Darstellung von N,N'-Di-(3-Nitratopivaloyl)-L-cystin-di-(N-Methylamid)

72,4 g (0,1 Mol) N,N'-Di-(3-Nitratopivaloyl)-L-cystin-di-(N-hydroxysuccinimidester) (Beispiel 13, 1. Stufe) wurden in 300 ml Ethylacetat gelöst, unter Rühren bei Raumtemperatur eine Lösung, bestehend aus 19 ml (40 %iger) N-Methylaminlösung und 60 ml Wasser, zugetropft und 15 Min. nachgerührt. Hierauf wurden nochmals 1,7 ml zugegeben und 30 Min. nachgerührt.

Die organische Phase wurde abgetrennt, mit 100 ml Wasser gewaschen, mit Natriumsulfat getrocknet und mit Aktivkohle geklärt. Das Lösungsmittel wurde vollständig abgezogen, wobei 59,44 g Rückstand verblieben. Dieser wurde in 90 ml Methanol gelöst, mit 40 ml Wasser versetzt und umkristallisiert.

Das ausgefallene Produkt wurde abgesaugt und zweimal mit je 25 ml Methanol/Wasser 1:1 gewaschen.

Die Ausbeute betrug 38,51 g (entsprechend 69,18 % d. Th.).
Fp: 166,5°C

### Beispiel 15

### Darstellung von N,N'-Di-(3-Nitratopivaloyl)-L-cystin-di-(N-butylamid)

7,3 g (0,01 Mol) N,N'-Di(3-Nitratopivaloyl)-L-cystin-di-(N-hydroxysuccinimidester) (Beispiel 13, 1. Stufe) wurden in 80 ml Dichlormethan bei Raumtemperatur gelöst, unter Rühren eine Lösung von 1,61 g (0,022 Mol) n-Butylamin in 20 ml Dichlormethan zugegeben und eine Stunde nachgerührt. Anschließend wurde die organische Phase nacheinander je einmal mit 10 ml 1 n Salzsäure, 10 ml 9 %iger Natriumhydrogencarbonat-Lösung und 10 ml Wasser gewaschen und am Rotavapor® abdestilliert, wobei 5,7 g (= 89,0 % d.Th.) Rückstand erhalten wurde, der aus 20 ml Methanol und 2 ml H₂O bei 0°C umkristallisiert wurde.

Die Ausbeute betrug 4,3 g (entsprechend 67,1 % d.Th.).
Fp: 78,4°C

### Beispiel 16

### Darstellung von N,N'-Di-(3-Nitratopivaloyl)-L-cystin-di-(N-tert.-butylamid)

7,3 g (0,01 Mol) N,N'-(3-Nitratopivaloyl)-L-cystin-di-(N-hydroxysuccinimidester) (Beispiel 13, 1. Stufe) wurden in 80 ml Dichlormethan gelöst, unter Rühren eine Lösung von 1,61 g (0,022 Mol) tert.-Butylamin in 20 ml Dichlormethan zugegeben und eine Stunde nachgerührt. Anschließend wurde die organische Phase nacheinander je einmal mit 10 ml 1 n Salzsäure, 10 ml 9 %iger Natriumhydrogencarbonat-Lösung und 10 ml Wasser gewaschen und am Rotavapor® abdestilliert, wobei 6,1 g (= 95,2 % d.Th.) Rückstand erhalten wurde, der aus 25 ml Methanol und 2 ml H₂O bei 0°C umkristallisiert wurde.

Die Ausbeute betrug 2,4 g (entsprechend 37,5 % d.Th.).
Fp: 81-82°C

### Beispiel 17

### Darstellung von N,N'-Di-(3-Nitratopivaloyl)-L-cystindimorpholid

7,3 g (0,01 Mol) N,N-Di(3-Nitratopivaloyl)-L-cystin-di-(N-hydroxysuccinimidester) (Beispiel 13, 1. Stufe) wurde in 80 ml Dichlormethan gelöst, unter Rühren eine Lösung von 1,92 g (0,022 Mol) Morpholin in 20 ml Dichlormethan zugetropft und über Nacht bei Raumtemperatur gerührt. Anschließend wurde die organische Phase nacheinander mit jeweils 10 ml 1 n Salzsäure, konzentrierte Natriumhydrogencarbonatlösung und Wasser gewaschen, mit Natriumsulfat getrocknet, filtriert und vollständig abdestilliert, wobei 6,06 g (entsprechend 91,2 % d.Th.) Rückstand verblieben, der säulenchromatographisch aufgereinigt wurde.

Die Ausbeute betrug 4,71 g (entsprechend 70,9 % d.Th.).
Fp: 33,6 °C

### Beispiel 18

### Darstellung von N,N'-Di-(3-Nitratopivaloyl)-L-cystindiisopropylester

7,3 g (10 mMol) N,N'-Di-(3-Nitratopivaloyl)-L-cystin-di-(N-hydroxysuccinimidester) (Beispiel 13, 1. Stufe) wurde in 150 ml Propanol-2 gelöst, über Nacht bei Raumtemperatur gerührt und das Propanol-2 anschließend am Rotavapor® weitgehend abgezogen. Der verbliebene Rückstand wurde in 50 ml Dichlormethan aufgenommen, zweimal mit jeweils 25 ml Wasser gewaschen, über Natriumsulfat getrocknet und am Rotationsverdampfer vollständig einrotiert, wobei ein Rückstand von 5,86 g Öl (95,28 % d.Th.) verblieb.

Dieser wurde unter Erwärmen in 35 ml Methanol gelöst und bei -25°C umkristallisiert. Das ausgefallene Produkt wurde abgesaugt, zweimal mit jeweils 5 ml tiefgekühltem Methanol gewaschen und im Vakuumtrockenschrank bei Raumtemperatur getrocknet.

Die Ausbeute betrug 4,01 g (entsprechend 65,31 % d.Th.).
Fp: 88,2°C

### Beispiel 19

### Darstellung von N'-3-Nitratopivaloyl-L-cysteinamid-glutathionmixed-disulfid

3,1 g (10 mmol) Glutathion wurden in 10 ml H₂O gelöst und mit einer Lösung, bestehend aus 5,3 g (10 mmol) N,N'-Di-(3-Nitratopivaloyl)-L-cysteindiamid (Beispiel 13) in 50 ml Methanol unter Stickstoffbegasung vermischt, drei Tage bei Raumtemperatur stehengelassen und anschließend am Rotavapor® bis zur Trockene eingeengt. Der Rückstand von 8,7 g wurde in 30 ml Methanol und 20 ml Wasser gelöst und mittels Büchi Chromatographie System über eine RP-18 Säule chromatographiert. Die zweite Fraktion wurde am Rotavapor® bis zur Trockene eingeengt, wobei ein Rückstand von 1,5 g erhalten wurde, welcher unter Erwärmen in 1 ml Wasser und 25 ml Ethanol gelöst wurde. Die über Nacht im Kühlschrank bei 0°C auskristallierte Substanz wurde abgesaugt, zweimal mit 5 ml Ethanol von 0°C gewaschen und über Phosphopentoxid im Vakuumtrockenschrank bei Raumtemperatur und ca. 2 Torr bis zur Gewichtskonstanz getrocknet.

Die Ausbeute betrug 0,87 g (entsprechend 15,3 % d.Th.).
Fp: 87,7°C

### Beispiel 20

### Darstellung von N'-3-Nitratopivaloyl-L-cysteinethylesterglutathion-mixed-disulfid

17,7 g (60 mmol) 3-Nitratopivaloyl-L-cysteinethylester wurden in 100 ml Essigsäure gelöst und mit einer Lösung, bestehend aus 18,50 g (60 mmol) Glutathion in 85 ml H₂O und 50 ml Essigsäure versetzt. Zu dieser Mischung wurde unter Rühren und Wasserbadkühlung (10°C) eine wäßrige Lösung, bestehend aus 4,28 g (20 mmol) Kaliumjodat in 65 ml Wasser in ca. 30 Min. so zugetropft, bis der Ansatz eine hellgelbe Farbe hatte (exotherm). Nach einigen Min. Rühren bei Raumtemperatur kristallisierte eine Substanz, die mittels Dünnschichtchromatographie als N,N'-Di-(3-Nitratopivaloyl)-L-cystindiethylester identifiziert wurde. Die Lösung wurde mit 100 ml Wasser versetzt und auf ca. 0°C gekühlt, die auskristallisierte Substanz abgesaugt, mit 75 ml Wasser gewaschen und getrocknet (9,0 g = 76,7 % d.Th.). Die Mutterlauge hiervon wurde am Rotavapor® bis zur Trockene eingeengt und der zurückbleibende Rückstand mit 100 ml Wasser und 250 ml Ethylacetat verrührt. Hierbei fiel ein geleeartiges Produkt aus, welches sich nicht absaugen ließ und daher durch Zugabe von 100 ml 1 n HCl gelöst wurde. Die vereinigten wäßrigen Lösungen wurden am Rotavapor® bis zur Trockene eingeengt, wobei ein öliger Rückstand erhalten wurde (15,5 g). Dieser wurde in 50 ml Wasser gelöst und mit 25 %iger Ammoniaklösung auf einen pH-Wert von 4,5 eingestellt. Die eingestellte Lösung wurde auf 0°C gekühlt, wobei jedoch keine Kristallisation erfolgte. Sie wurde daher erneut am Rotavapor® bis zur Trockene eingeengt und mittels Büchi Chromatographiesystem über eine RP-18 Säule chromatographiert. Da keine Auftrennung erfolgte, wurde die Lösung eingeengt und der erhaltene Rückstand (10,9 g) mittels Abimed Chromatographiesystem in 15 Säulenläufen aufgetrennt. Die vereinigten zweiten Fraktionen wurden am Rotavapor® bis zur Trockene eingeengt, der verbliebene Rückstand (1,2 g) in 25 ml Ethanol und 1,5 ml Wasser unter Erwärmen gelöst und über Nacht bei 0°C in den Kühlschrank gestellt. Das dabei auskristallisierte Produkt wurde abgesaugt, zweimal mit 5 ml Ethanol von 0°C gewaschen und im Vakuumtrockenschrank bei Raumtemperatur und ca. 2 Torr über Phosphopentoxid bis zur Gewichtskonstanz getrocknet.

Die Ausbeute betrug 0,77 g (entsprechend 6,4 % d.Th.).
Fp: 167,6°C

### Beispiel 21

### Darstellung von N-3-Nitratopivaloyl-L-cysteinethylester-N'-acetyl-L-cystein-mixed-disulfid

5,9 g (20 mmol) 3-Nitratopivaloyl-L-cysteinethylester wurden in 50 ml Essigsäure gelöst und mit einer Lösung, bestehend aus 3,3 g (20 mmol) N-Acetyl-L-Cystein in 25 ml Wasser versetzt. Zu dieser Mischung wurde unter Rühren und Wasserbadkühlung (10°C) eine wäßrige Lösung, bestehend aus 1,43 g (0,66 mmol) Kaliumjodat in 25 ml Wasser in ca. 15 Min. so zugetropft, bis der Ansatz eine hellgelbe Farbe hatte (exotherm). Nach einigen Min. Rühren bei Raumtemperatur kristallisierte eine Substanz, die mittels Dünnschichtchromatographie als N,N'-Di-(3-Nitratopivaloyl)-L-cystindiethylester identifiziert wurde. Die Lösung wurde mit 100 ml Wasser versetzt und auf ca. 0°C gekühlt, die auskristallisierte Substanz abgesaugt und getrocknet (2,8 g = 71,6 % d.Th.). Die Mutterlauge hiervon wurde am Rotavapor® bis zur Trockene eingeengt. Der zurückbleibende Rückstand wurde mit 50 ml Wasser und 200 ml Ethylacetat verrührt. Die org. Phase wurde abgetrennt, nacheinander je einmal mit 50 ml 1 n HCl und 50 ml H₂O gewaschen, über Natriumsulfat getrocknet und am Rotavapor® bis zur Trockene eingeengt. Hierbei blieben 4,1 g öliges Produkt zurück, welches säulenchromatographisch mittels RP-Säule über Büchi System gereinigt wurde. Der Rückstand, der am Rotavapor® eingeengten zweiten Fraktion (3,9 g) wurde in 10 ml Methanol gelöst und mit 15 ml Wasser versetzt und unter Rühren auf ca. 0°C abgekühlt. Die dabei auskristallisierte Substanz wurde abgesaugt und im Vakuumtrockenschrank bis zur Gewichtskonstanz getrocknet.

Die Ausbeute betrug 2,4 g (entsprechend 79,0 % d.Th.).
Fp: Kristalle, nach einigen Wochen zähes Öl.

### Beispiel 22

### Darstellung von N-(3-Nitratopivaloyl)-L-cysteinethylester-D,L-penicillamin-mixed-disulfid

8,82 g (30 mmol) 3-Nitratopivaloyl-L-cysteinethylester wurden in 120 ml Methanol gelöst und bei 5°C - 10°C eine Lösung, bestehend aus 4,50 g (30 mmol) D,L-Penicillamin in 40 ml Wasser und 20 ml 1 n Salzsäure zugefügt. Nach Zugabe von 2,14 g (10 mmol) Kaliumjodat wurde ca. 1,5 h bei 10°C - 15°C gerührt, mit Natriumthiosulfat entfärbt und der kristallisierte Rückstand bei ca. 5°C abgesaugt. Nach Trocknen an der Luft wurden 5,8 g Rückstand erhalten, der dünnschichtchromatographisch als N,N'-Di-(3-Nitratopivaloyl)-L-cystindiethylester identifiziert wurde.

Das Filtrat wurde am Rotationsverdampfer vollständig einrotiert, der Rückstand in 100 ml konz. Natriumacetatlösung aufgenommen, bei ca. 80°C mit 1 g Aktivkohle geklärt und mit 20 g Natriumchlorid versetzt (klare Lösung). Das über Nacht im Kühlschrank kristallisierte Produkt wurde abgesaugt und bei Raumtemperatur im Vakuumtrockenschrank getrocknet.

Die Ausbeute betrug 1,83 g (entsprechend 13,8 % d.Th.)
Fp: 132,5°C

### Beispiel 23

### Darstellung von 2-Acetylamino-3-[2-(2,2-dimethyl-3-nitrooxypropionylamino)-2-ethoxycarbonyl-ethyldisulfanyl]-3-methyl-buttersäure

1,91 g (10 mmol) N-Acetyl-D,L-penicillamin und 2,94 g (10 mmol) 3-Nitratopivaloyl-L-cysteinethylester wurden in 40 ml Methanol und 15 ml Wasser gelöst. Bei 15°C - 20°C wurden portionsweise 710 mg (3,3 mmol) Kaliumjodat zugegeben. (Nach Zugabe einer zusätzlichen Spatelspitze Kaliumjodat blieb eine dauerhafte Gelbfärbung durch freies Jod.) Der Ansatz wurde durch Zugabe von wenig Natriumthiosulfat entfärbt und einrotiert. Der Rückstand wurde in konz. Natriumhydrogencarbonatlösung aufgenommen (CO₂-Entwicklung) und das N,N'-Di-(3-Nitratopivaloyl)-L-cystindiethylester unter DC-Kontrolle durch mehrmaliges Extrahieren mit Dichlormethan entfernt. Die Extrakte wurden einrotiert, wobei ein Rückstand von 1,1 g verblieb.

Die wäßrige Phase wurde mit konz. Salzsäure angesäuert und mit 50 ml Ethylacetat extrahiert. Das Ethylacetat wurde vollständig abgezogen, wobei ein Rückstand von 1,48 g verblieb.

Der Gesamtrückstand wurde in 150 mg-Portionen säulenchromatographisch gereinigt (Abimed Chromatographiesystem, RP-18 Säule).

Der Rückstand aus Fraktion 2 wurde aus 30 ml Ethylacetat/n-Hexan 1/1 umkristallisiert.

Die Ausbeute betrug 350 mg (entsprechend 7,24 % d.Th.).
Fp: 120,2°C

### Pharmakologische Daten

### Gefäßrelaxation in vitro

Die gefäßerschlaffende Wirkung der Verbindungen der allgemeinen Formel I wurde in vitro an isolierten Rattenaortenringen untersucht. Hierzu wurde Ratten nach Betäubung die Aorta thoracalis entnommen, diese von Fett- und Adventivgewebe befreit und in ca. 5 mm breite Ringsegmente zerteilt. Die Aortenringe wurden in temperierte, mit Carbogen begaste Bäder mit Tyrodelösung überführt und einer Vorlast von 2 g ausgesetzt. Unter kontinuierlicher Registrierung der Kraftentwicklung wurden die Gefäßringe nach 90 minütiger Äquilibrierung mit 2 x 10⁻⁷ mol/l Phenylephrin vorkontrahiert. Nach Erreichen eines stabilen Kontraktionsniveaus wurden die Nitratoverbindungen kumulativ der Gefäßbadflüssigkeit zugesetzt, wobei die Zugabe der jeweiligen nächsthöheren Konzentration erst nach Erreichen eines stabilen Relaxationsniveaus erfolgte. Aus den erhaltenen Konzentrations-Wirkungs-Kurven wurde die Konzentration der jeweiligen Verbindung ermittelt, welche zu einer 50 %igen Relaxation des vorkontrahierten Gefäßringes führt (EC₅₀-Wert). Tabelle 1 enthält die so ermittelten EC₅₀-Werte beispielhaft ausgewählter Verbindungen.

**Tabelle 1**

| Substanz | EC₅₀ [mol/l] | Anzahl der Einzelversuche [n] |
|---|---|---|
| N,N'-Di-(3-Nitratopivaloyl)-L-cystindiethylester | 1,5 x 10⁻⁶ | 6 |
| N,N'-Di-(3-Nitratopivaloyl)-L-cystin | 1,2 x 10⁻⁴ | 6 |
| N,N'-Di-(3-Nitratopivaloyl)-L-cystindiamid | 3,0 x 10⁻⁵ | 6 |
| N,N'-Di-(3-Nitratopivaloyl)-L-cystin-di-tert.-butylester | 5,9 x 10⁻⁵ | 3 |
| N,N'-Di-(3-Nitratopivaloyl)-L-cystindipiperidid | 1,0 x 10⁻⁴ | 6 |
| N,N'-Di-(3-Nitratopivaloyl)-L-cystin-di-(N,N'-methylamid) | 2,4 x 10⁻⁵ | 4 |

### Blutdruckeffekte am wachen Hund

Zum Nachweis der Nitratwirkung in vivo wurden die Effekte der erfindungsgemäßen Verbindungen auf den systolischen arteriellen Blutdruck (SAP), zentralvenösen Blutdruck (CVP) und pulmonalarteriellen Blutdruck (PAP) am wachen Hund untersucht. Die Messung des SAP erfolgte über einen über die Femoralarterie in die Aorta eingeführten Tip-Katheter-Transducer, die Messung des CVP sowie des PAP über einen über die V.jugularis sin. eingeführten Swan-Ganz-Katheter, dessen Katheterspitzen bis zur A. pulmonalis bzw. bis zum rechten Herzvorhof vorgeschoben wurden, über deren Lumen der jeweilige Druck auf Statham-Druckumwandler gegeben wurde. Zur Quantifizierung der durch die erfindungsgemäße Verbindung hervorgerufenen Blutdrucksenkung wurden die Flächen unterhalb der registrierten Blutdruck-Verlaufskurven (AUC) bestimmt. Tabelle 2 enthält die AUC-Werte der jeweiligen Blutdrucksenkung nach intravenöser Gabe von 13,4 µg/kg der erfindungsgemäßen Disulfidverbindungen bzw. nach intravenöser Gabe von 26,8 µg/kg Isosorbid-5-mononitrat (5-ISMN).

**Tabelle 2**

| | AUC (SAP) (mg) | AUC (CVP) (mg) | AUC (PAP) (mg) |
|---|---|---|---|
| N,N'-Di-(3-Nitratopivaloyl)-L-cystindiethylester | 94,1 | 85,7 | 12,0 |
| N,N'-Di-(3-Nitratopivaloyl)-L-cystin | 17,3 | 67,9 | 2,7 |
| N,N'-Di-(3-Nitratopivaloyl)-L-cystindiamid | 55,3 | 46,9 | 28,1 |
| N,N'-Di-(3-Nitratopivaloyl)-L-cystindipiperidid | 10,4 | 36,9 | 18,5 |
| N,N'-Di-(3-Nitratopivaloyl)-L-cystin-di-(N,N'-methylamid) | 41,9 | 33,7 | 3,5 |
| 5-ISMN | 22,2 | 25,4 | 8,9 |

Die gegenüber dem klassischen Nitrat 5-ISMN deutlich erhöhten AUC-Werte für die Blutdrucksenkung nach Gabe der erfindungsgemäßen Verbindungen belegen ihre hohe Wirksamkeit.

## Patentansprüche

1. Verbindungen der allgemeinen Formel I 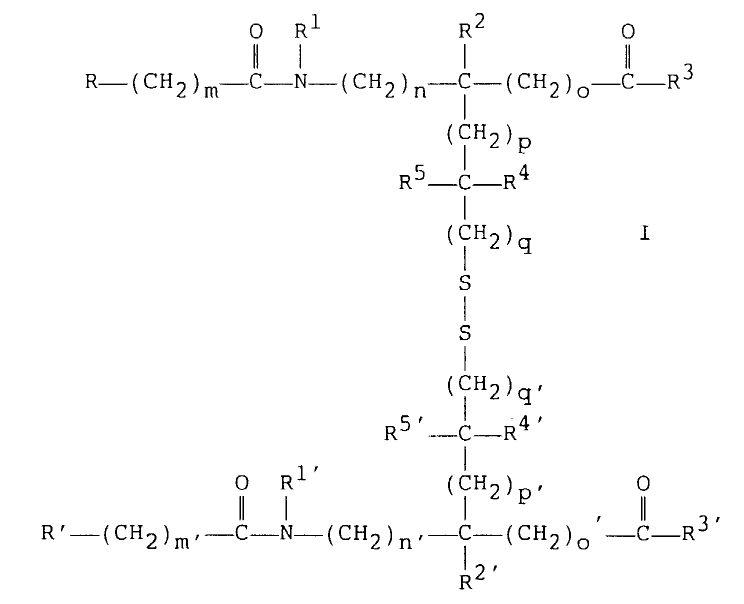 in der R und R' Gruppen der allgemeinen Formeln 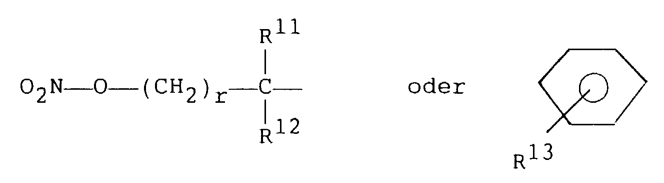 bedeuten, worin
R¹¹ Wasserstoff, Alkyl mit 1 bis 6 Kohlenstoffatomen, substituiertes C₁-C₆-Niedrigalkyl, worin der Substituent Halogen, Hydroxy, C₁-C₆-Niedrigalkoxy, Aryloxy, Amino, C₁-C₆-Niedrigalkylamino, Acylamino, Acyloxy, Arylamino, Mercapto, C₁-C₆-Niedrigalkylthio, Arylthio ist
R¹² Wasserstoff oder C₁-C₆-Niedrigalkyl bedeuten
R¹³ Nitratoalkyl mit 1 bis 6 Kohlenstoffatomen ist
r die Zahlenwerte 0 bis 10 besitzt
und in der
R¹, R^{1'}, Wasserstoff oder C₁-C₆-Niedrigalkyl bedeuten
R², R^{2'} Wasserstoff, C₁-C₆-Niedrigalkyl, Phenyl, Methoxyphenyl, Phenyl-C₁-C₆-niedrigalkyl, Methoxyphenyl-C₁-C₆-niedrigalkyl, Hydroxyphenyl-C₁-C₆-niedrigalkyl, Hydroxy-C₁-C₆-niedrigalkyl, Alkoxy-C₁-C₆-niedrigalkyl, Amino-C₁-C₆-niedrigalkyl, Acylamino-C₁-C₆-niedrigalkyl, Mercapto-C₁-C₆-niedrigalkyl oder C₁-C₆-Niedrigalkylthio-C₁-C₆-niedrigalkyl ist
R³, R^{3'} Hydroxy, C₁-C₆-Niedrigalkoxy, C₁-C₆-Niedrigalkenoxy, Di-C₁-C₆-niedrigalkylamino-C₁-C₆-niedrigalkoxy, Acylamino-C₁-C₆-niedrigalkoxy, Acyloxy-C₁-C₆-niedrigalkoxy, Aryloxy, Aryl-C₁-C₆-niedrigalkoxy, substituiertes Aryloxy oder substituiertes Aryl-C₁-C₆-niedrigalkoxy, worin der Substituent Methyl, Halogen oder Methoxy ist; Amino, C₁-C₆-Niedrigalkylamino, Di-C₁-C₆-niedrigalkylamino, Aryl-C₁-C₆-niedrigalkylamino, Hydroxy-C₁-C₆-niedrigalkylamino, Pyrrolidin, Piperidin, Morpholin, Piperazin oder Aminosäurereste über Peptidverknüpfung sind
R⁴, R^{4'} Wasserstoff oder C₁-C₆-Niedrigalkyl ist,
R⁵, R^{5'} wie R⁴, R^{4'} Wasserstoff oder C₁-C₆-Niedrigalkyl bedeuten,
R² und R³ sowie R^{2'} und R^{3'} miteinander unter Bildung eines Esters oder Amids verbunden sein können,
R¹ und R² sowie R^{1'} und R^{2'} miteinander unter Bildung einer Alkylenbrücke mit 2 bis 4 Kohlenstoffatomen, einer Alkylenbrücke mit 2 bis 3 Kohlenstoffatomen und einem Schwefelatom, einer Alkylenbrücke mit 3 bis 4 Kohlenstoffatomen, die eine Doppelbindung oder eine Alkylenbrücke wie oben enthält, substituiert durch Hydroxy, C₁-C₆-Niedrigalkoxy, C₁-C₆-Niedrigalkyl oder Di-C₁-C₆-niedrigalkyl, verbunden sein können,
m, n, o, p, q sowie m', n', o', p' und q' die Zahlenwerte 0 bis 10 besitzen
sowie deren physiologisch verträgliche Salze.

2. Verbindungen nach Anspruch 1, dadurch gekennzeichnet, daß deren Nitratoalkan- oder Nitratoalkylarylalkan-Carbonsäure-Bestandteile eine Kettenlänge von 2 bis 6 Kohlenstoffatomen haben, die geradkettig, verzweigt, racemisch oder optisch isomer sind.

3. Verbindungen nach Ansprüchen 1 und 2, dadurch gekennzeichnet, daß die in ihnen enthaltenen Aminosäuredisulfide Cystin, Homocystin oder Penicillamindisulfid sind.

4. Verbindungen nach Ansprüchen 1 bis 3, dadurch gekennzeichnet, daß die Aminosäuredisulfide in der stereochemischen L-Form vorliegen.

5. Verbindungen nach Ansprüchen 1 bis 3, dadurch gekennzeichnet, daß die Aminosäuredisulfide in der stereochemischen D,L-Form vorliegen.

6. Verbindungen nach Ansprüchen 1 bis 5, dadurch gekennzeichnet, daß sie die folgenden chemischen Formeln aufweisen
N,N'-Di-(3-Nitratopivaloyl)-L-cystin
N,N'-Di-(3-Nitratopivaloyl)-D,L-Homocystin
N,N'-Di-(3-Nitratopivaloyl)-L-cystin-diethylester
N,N'-Di-(3-Nitratopivaloyl)-D,L-Homocystin-diethylester
N,N'-Di-(3-Nitratopivaloyl)-L-cystin-di-(2-Methyl-2 Propylester)
N,N'-Di-(4-Nitratomethylbenzoyl)-L-cystin-dimethylester
N,N'-Di-(3-Nitratomethylbenzoyl)-L-cystin-dimethylester
N,N'-Di-(4-Nitratomethylbenzoyl)-L-cystin-di-(N,N'-butylamid)
N,N'-Di-(3-Nitratomethylbenzoyl)-L-cystin-di-(N,N'-butylamid)
N,N'-Di-(4-Nitratomethylbenzoyl)-L-cystindiamid
N,N'-Di-(3-Nitratomethylbenzoyl)-L-cystindiamid
N,N'-Di-(3-Nitratopivaloyl)-L-penicillamin-disulfid-diamid
N,N'-Di-(3-Nitratopivaloyl)-L-cystin-diamid
N,N'-Di-(3-Nitratopivaloyl)-L-cystin-di-(N,N'-methylamid)
N,N'-Di-(3-Nitratopivaloyl)-L-cystin-di-(N,N'-butylamid)
N,N'-Di-(3-Nitratopivaloyl)-L-cystin-di-(N,N'-tert.-butylamid)
N,N'-Di-(3-Nitratopivaloyl)-L-cystin-di-morpholid
N,N'-Di-(3-Nitratopivaloyl)-L-cystin-di-isopropylester

7. Verfahren zur Herstellung von Verbindungen gemäß den Ansprüchen 1 bis 6, dadurch gekennzeichnet, daß die Verbindungen in an sich bekannter Weise durch Kondensation der entsprechenden Nitratoalkyl- oder Nitratoalkylarylalkylcarbonsäure oder deren reaktionsfähigen Derivaten mit den Aminogruppen eines Aminosäuredisulfids umgesetzt werden.

8. Verfahren zur Herstellung von Verbindungen gemäß den Ansprüchen 1 bis 6, dadurch gekennzeichnet, daß die entsprechenden Nitratoalkyl- oder Nitratoalkylarylalkyl-Carbonsäuren oder deren reaktive Derivate mit der Aminogruppe von Thiolgruppen enthaltenden Aminosäuren kondensiert und die erhaltenen Verbindungen anschließend unter Dimerisierung zu den entsprechenden Disulfiden oxidiert werden.

9. Verfahren zur Herstellung von Verbindungen der allgemeinen Formel II 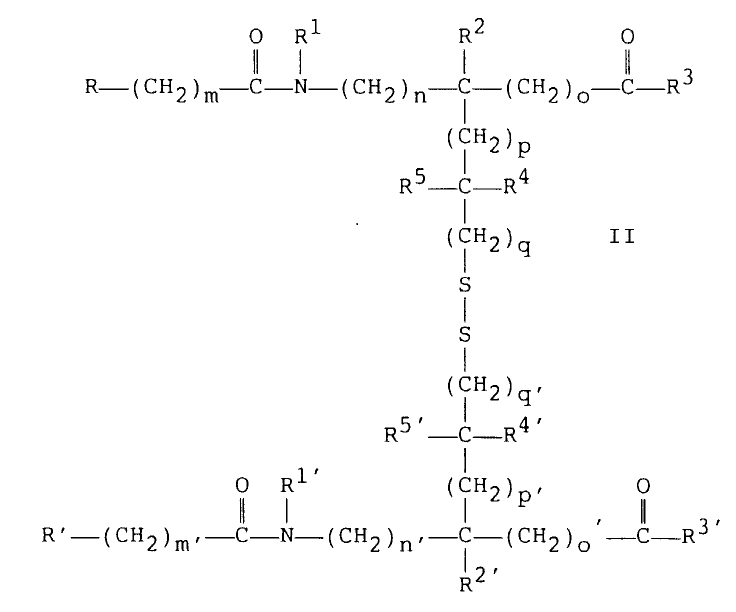 in der R und R' Gruppen der allgemeinen Formeln 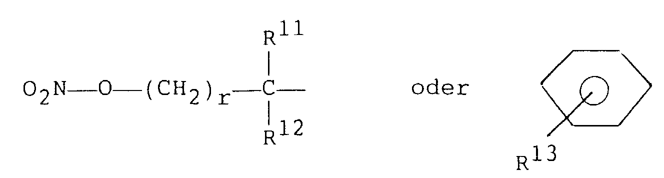 bedeuten, worin
R¹¹ Wasserstoff, Alkyl mit 1 bis 6 Kohlenstoffatomen, substituiertes C₁-C₆-Niedrigalkyl, worin der Substituent Halogen, Hydroxy, C₁-C₆-Niedrigalkoxy, Aryloxy, Amino, C₁-C₆-Niedrigalkylamino, Acylamino, Acyloxy, Arylamino, Mercapto, C₁-C₆-Niedrigalkylthio, Arylthio ist
R¹² Wasserstoff oder C₁-C₆-Niedrigalkyl bedeuten
R¹³ Nitratoalkyl mit 1 bis 6 Kohlenstoffatomen ist
r die Zahlenwerte 0 bis 10 besitzt
und in der
R¹, R^{1'}, Wasserstoff oder C₁-C₆-Niedrigalkyl bedeuten
R², R^{2'} Wasserstoff, C₁-C₆-Niedrigalkyl, Phenyl, Methoxy-phenyl, Phenyl-C₁-C₆-niedrigalkyl, Methoxy-phenyl-C₁-C₆-niedrigalkyl, Hydroxyphenyl-C₁-C₆-niedrigalkyl, Hydroxy-C₁-C₆-niedrigalkyl, Alkoxy-C₁-C₆-niedrigalkyl, Amino-C₁-C₆-niedrigalkyl, Acylamino-C₁-C₆-niedrigalkyl, Mercapto-C₁-C₆-niedrigalkyl oder C₁-C₆-Niedrigalkylthio-C₁-C₆-niedrigalkyl ist
R³, R^{3'} Amino, C₁-C₆-Niedrigalkylamino, Di-C₁-C₆-niedrigalkylamino, Aryl-C₁-C₆-niedrigalkylamino, Hydroxy-C₁-C₆-niedrigalkylamino, Pyrrolidin, Piperidin, Morpholin, Piperazin oder Aminosäurereste über Peptidverknüpfung sind
R⁴, R^{4'} Wasserstoff oder C₁-C₆-Niedrigalkyl ist,
R⁵, R^{5'} wie R⁴, R^{4'} Wasserstoff oder C₁-C₆-Niedrigalkyl bedeuten,
R¹ und R² sowie R^{1'} und R^{2'} miteinander unter Bildung einer Alkylenbrücke mit 2 bis 4 Kohlenstoffatomen, einer Alkylenbrücke mit 2 bis 3 Kohlenstoffatomen und einem Schwefelatom, einer Alkylenbrücke mit 3 bis 4 Kohlenstoffatomen, die eine Doppelbindung oder eine Alkylenbrücke wie oben enthält, substituiert durch Hydroxy, C₁-C₆-Niedrigalkoxy, C₁-C₆-Niedrigalkyl oder Di-C₁-C₆-niedrigalkyl, verbunden sein können,
m, n, o, p, q sowie m', n', o', p' und q' die Zahlenwerte 0 bis 10 besitzen,
das dadurch gekennzeichnet ist, daß Verbindungen der allgemeinen Formel III 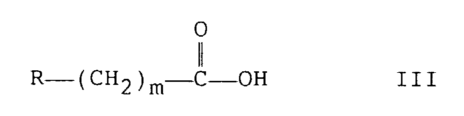 in der R eine Gruppe der allgemeinen Formel 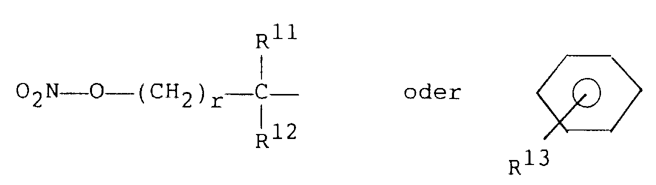 bedeutet, worin
R¹¹ Wasserstoff, Alkyl mit 1 bis 6 Kohlenstoffatomen, substituiertes C₁-C₆-Niedrigalkyl, worin der Substituent Halogen, Hydroxy, C₁-C₆-Niedrigalkoxy, Aryloxy, Amino, C₁-C₆-Niedrigalkylamino, Acylamino, Acyloxy, Arylamino, Mercapto, C₁-C₆-Niedrigalkylthio, Arylthio ist
R¹² Wasserstoff oder C₁-C₆-Niedrigalkyl bedeuten
R¹³ Nitratoalkyl mit 1 bis 6 Kohlenstoffatomen ist
m und r die Zahlenwerte 0 - 10 besitzen
in Form ihrer freien Säuren, reaktiven Säurehalogenide, Säure-Azide, Ester und Säure-Anhydride in an sich bekannter Weise mit Verbindungen der allgemeinen Formel IV 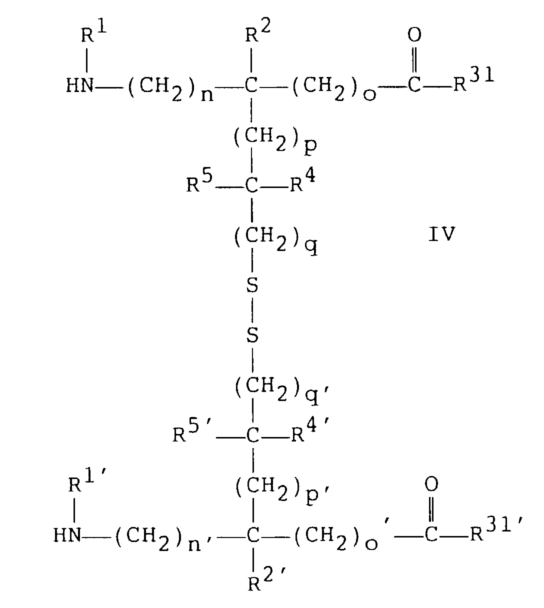 in denen
R¹, R^{1'} Wasserstoff oder C₁-C₆-Niedrigalkyl bedeuten
R², R^{2'} Wasserstoff, C₁-C₆-Niedrigalkyl, Phenyl, Methoxy-phenyl, Phenyl-C₁-C₆-niedrigalkyl, Methoxy-phenyl-C₁-C₆-niedrigalkyl, Hydroxyphenyl-C₁-C₆-niedrigalkyl, Hydroxy-C₁-C₆-niedrigalkyl, Alkoxy-C₁-C₆-niedrigalkyl, Amino-C₁-C₆-niedrigalkyl, Acylamino-C₁-C₆-niedrigalkyl, Mercapto-C₁-C₆-niedrigalkyl oder C₁-C₆-Niedrigalkylthio-C₁-C₆-niedrigalkyl ist
R³¹, R^{31'} Hydroxy oder Halogen bedeutet
R⁴, R^{4'} Wasserstoff oder C₁-C₆-Niedrigalkyl ist,
R⁵, R^{5'} wie R⁴, R^{4'} Wasserstoff oder C₁-C₆-Niedrigalkyl bedeuten,
R¹ und R² sowie R^{1'} und R^{2'} miteinander unter Bildung einer Alkylenbrücke mit 2 bis 4 Kohlenstoffatomen, einer Alkylenbrücke mit 2 bis 3 Kohlenstoffatomen und einem Schwefelatom, einer Alkylenbrücke mit 3 bis 4 Kohlenstoffatomen, die eine Doppelbindung oder eine Alkylenbrücke wie oben enthält, substituiert durch Hydroxy, C₁-C₆-Niedrigalkoxy, C₁-C₆-Niedrigalkyl oder Di-C₁-C₆-niedrigalkyl, verbunden sein können,
n, o, p, q sowie n', o', p' und q' die Zahlenwerte 0 bis 10 besitzen
zu Verbindungen der allgemeinen Formel V 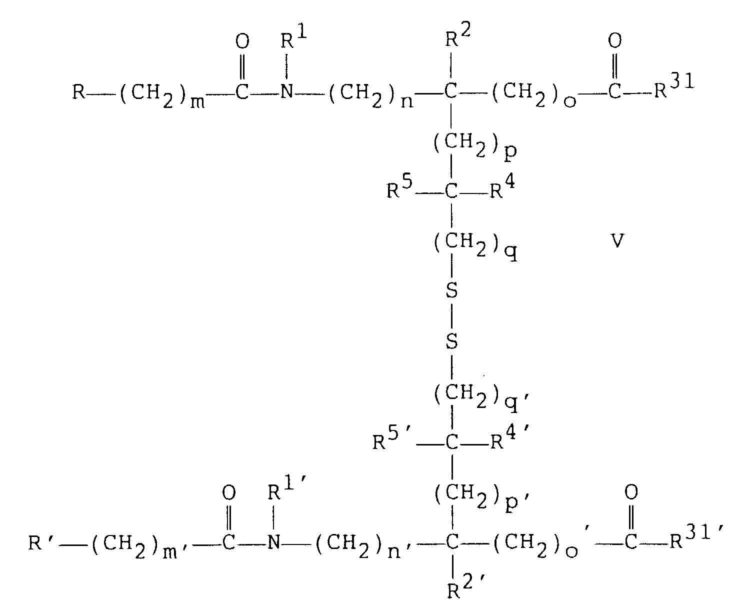 kondensiert werden, diese an ihrer freien oder halogenierten Carbonsäurefunktionen mit N-Hydroxysuccinimid zu aktivierten Carbonsäure-N-hydroxysuccinimidester der allgemeinen Formel VI 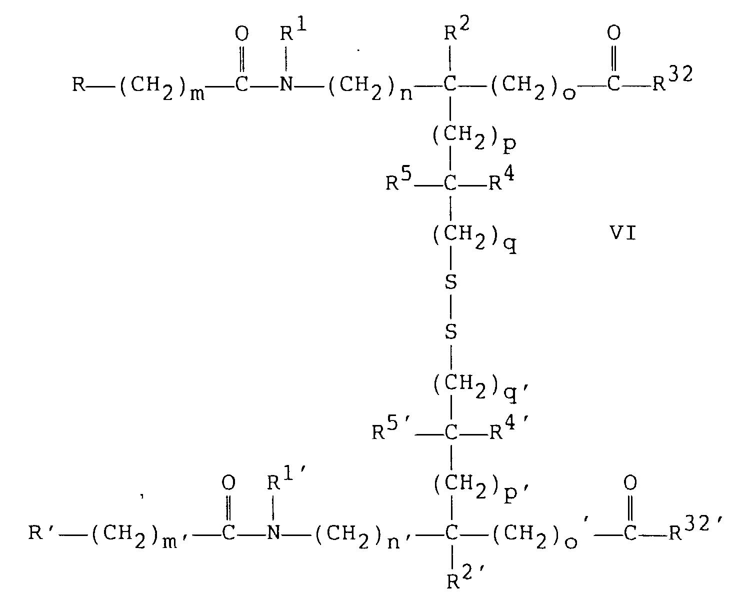 überführt werden,
in denen
R³² und R^{32'} 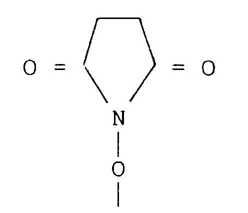
bedeutet
und diese anschließend mit Ammoniak, einem primären oder einem sekundären Amin, insbesondere mit C₁-C₆-Niedrigalkylamin, Di-C₁-C₆-niedrigalkylamin, Aryl-C₁-C₆-niedrigalkylamin, Hydroxy-C₁-C₆-niedrigalkylamin, Pyrrolidin, Piperidin, Morpholin, Piperazin oder Aminosäuren zu den Verbindungen der allgemeinen Formel II umgesetzt werden.

10. Verbindungen, die die folgenden Bezeichnungen tragen
- N'-3-Nitratopivaloyl-L-cysteinamid-glutathion-mixeddisulfid
- N'-3-Nitratopivaloyl-L-cysteinethylester-glutathionmixed-disulfid
- N-3-Nitratopivaloyl-L-cysteinethylester-N'-acetyl-L-cystein-mixed-disulfid
- N-(3-Nitratopivaloyl)-L-cysteinethylester-D,L-penicillamin-mixed-disulfid
- 2-Acetylamino-3-[2-(2,2-dimethyl-3-nitrooxy-propionylamino)-2-ethoxycarbonyl-ethyldisulfanyl]-3-methylbuttersäure

11. Arzneimittel enthaltend eine oder mehrere Verbindung/en gemäß Ansprüchen 1 bis 6 und 10 sowie übliche Träger- und Hilfsstoffe.

## Claims

1. Compounds of the general formula I 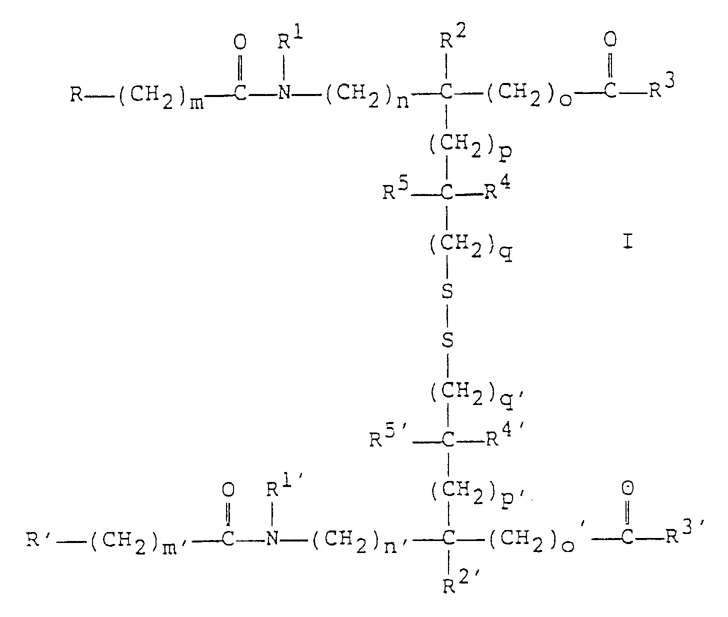 in which R and R' denote groups of the general formulae 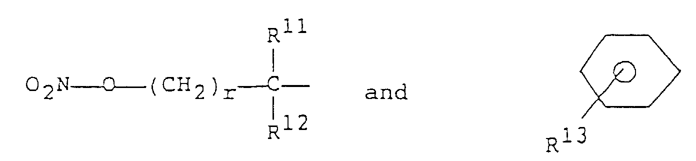 in which
R¹¹ denotes hydrogen, alkyl having 1 to 6 carbon atoms, substituted lower alkyl in which the substituent is halogen, hydroxyl, lower alkoxy having 1 to 6 carbon atoms, aryloxy, amino, lower alkylamino having 1 to 6 carbon atoms, acylamino, acyloxy, arylamino, mercapto, lower alkylthio having 1 to 6 carbon atoms, arylthio,
R¹² denotes hydrogen or lower alkyl having 1 to 6 carbon atoms,
R¹³ is nitratoalkyl having 1 to 6 carbon atoms,
r has the numerical values 0 to 10,
and in which
R¹, R^{1'} denote hydrogen or lower alkyl having 1 to 6 carbon atoms,
R², R^{2'} are hydrogen, lower alkyl having 1 to 6 carbon atoms, phenyl, methoxyphenyl, phenyl-lower-alkyl having 1 to 6 carbon atoms, methoxyphenyllower-alkyl having 1 to 6 carbon atoms, hydroxy-phenyl-lower-alkyl having 1 to 6 carbon atoms, hydroxylower-alkyl having 1 to 6 carbon atoms, alkoxy-lower-alkyl having 1 to 6 carbon atoms, amino-lower-alkyl having 1 to 6 carbon atoms, acylamino-lower-alkyl having 1 to 6 carbon atoms, mercapto-lower-alkyl having 1 to 6 carbon atoms or lower alkyl-thio-lower-alkyl having 1 to 6 carbon atoms,
R³, R^{3'} are hydroxyl, lower alkoxy having 1 to 6 carbon atoms, lower alkenoxy having 1 to 6 carbon atoms, di-loweralkylamino-lower-alkoxy having 1 to 6 carbon atoms, acylamino-lower-alkoxy having 1 to 6 carbon atoms, acyloxylower-alkoxy having 1 to 6 carbon atoms, aryloxy, aryl-lower-alkoxy having 1 to 6 carbon atoms, substituted aryloxy or substituted aryl-lower-alkoxy having 1 to 6 carbon atoms in which the substituent is methyl, halogen or methoxy; amino, lower alkylamino having 1 to 6 carbon atoms, di-loweralkylamino having 1 to 6 carbon atoms, aryl-lower-alkylamino having 1 to 6 carbon atoms, hydroxy-loweralkyl-amino having 1 to 6 carbon atoms, pyrrolidine, piperidine, morpholine, piperazine or amino-acid residues via peptide linkage,
R⁴, R^{4'} are hydrogen or lower alkyl having 1 to 6 carbon atoms,
R⁵, R^{5'} denote like R⁴, R^{4'} hydrogen or lower alkyl having 1 to 6 carbon atoms,
R² and R³, and R^{2'} and R^{3'}, can be linked together and R and R , can be linked together to form an ester or amide,
R¹ and R², and R^{1'} and R^{2'}, can be linked together to form an alkylene bridge having 2 to 4 carbon atoms, an alkylene bridge having 2 to 3 carbon atoms and a sulphur atom, an alkylene bridge having 3 to 4 carbon atoms which contains a double bond or an alkylene bridge as above, substituted by hydroxyl, lower alkoxy having 1 to 6 carbon atoms, lower alkyl having 1 to 6 carbon atoms or di-lower-alkyl having 1 to 6 carbon atoms,
m, n, o, p, q and m', n', o', p' and q' have the numerical values 0 to 10,
and the physiologically tolerated salts thereof.

2. Compounds according to Claim 1, characterized in that their nitratoalkane- or nitratoalkylarylalkanecarboxylic acid components have a chain length of 2 to 6 carbon atoms which are straight-chain, branched, racemic or optically isomeric.

3. Compounds according to Claims 1 and 2, characterized in that the amino-acid disulphides contained in them are cystine, homocystine or penicillamine disulphide.

4. Compounds according to Claims 1 to 3, characterized in that the amino-acid disulphides are in the stereochemical L-form.

5. Compounds according to Claims 1 to 4, characterized in that the amino-acid disulphides are in the stereochemical D,L-form.

6. Compounds according to Claims 1 to 5, characterized in that they have the following chemical formulae
N,N'-di(3-nitratopivaloyl)-L-cystine
N,N'-di(3-nitratopivaloyl)-D,L-homocystine
N,N'-di(3-nitratopivaloyl)-L-cystine diethyl ester
N,N'-di(3-nitratopivaloyl)-D,L-homocystine diethyl ester
N,N'-di(3-nitratopivaloyl)-L-cystine-di-(2-methyl-2-propylester)
N,N'-di(4-nitratomethylbenzoyl)-L-cystine dimethyl ester
N,N'-di(3-nitratomethylbenzoyl)-L-cystine dimethyl ester
N,N'-di(4-nitratomethylbenzoyl)-L-cystine-di(N,N'-butyl-amide)
N,N'-di(3-nitratomethylbenzoyl)-L-cystine-di(N,N'-butyl-amide)
N,N'-di(4-nitratomethylbenzoyl)-L-cystinediamide
N,N'-di(3-nitratomethylbenzoyl)-L-cystinediamide
N,N'-di(3-nitratopivaloyl)-L-penicillamine disulphide-diamide
N,N'-di(3-nitratopivaloyl)-L-cystinediamide
N,N'-di(3-nitratopivaloyl)-L-cystine-di(N,N-methylamide)
N,N'-di(3-nitratopivaloyl)-L-cystine-di((N,N-butylamide)
N,N'-di(3-nitratopivaloyl)-L-cystine-di(N,N-tert.-butylamide)
N,N'-di(3-nitratopivaloyl)-L-cystine-dimorpholide
N,N'-di(3-nitratopivaloyl)-L-cystinediisopropyl ester.

7. Process for the preparation of compounds according to Claims 1 to 6, characterized in that the compounds are obtained in a manner known per se by condensation of the appropriate nitratoalkyl- or nitratoalkylarylalkylcarboxlic acid or its reactive derivatives with the amino groups of an amino-acid sulphide.

8. Process for the preparation of compounds according to Claims 1 to 6, characterized in that the appropriate nitratoalkyl- or nitraoalkylarylalkylcarboxylic acids or their reactive derivatives are condensed with the amino group of amino acids containing thiol groups, and the resulting compounds are subsequently oxidized with dimerization to the corresponding disulphides.

9. Process for the preparation of compounds of the general formula II 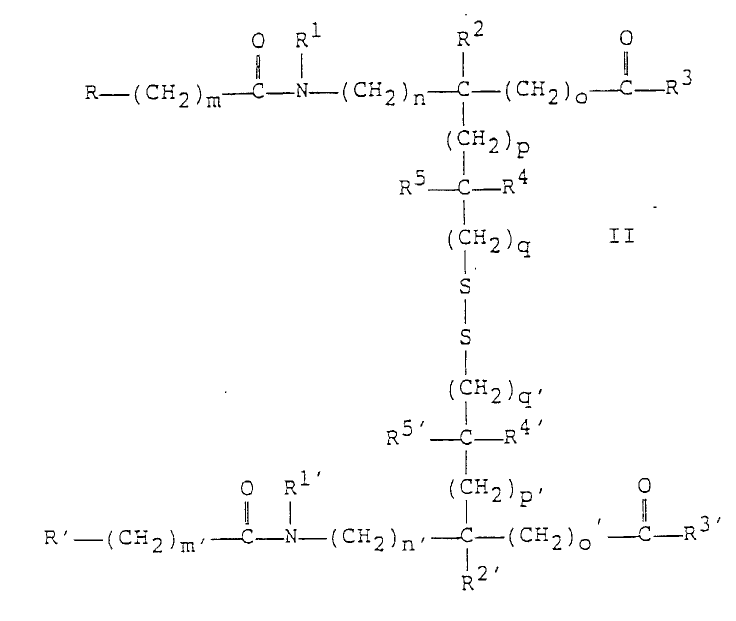 in which R and R' denote groups of the general formulae 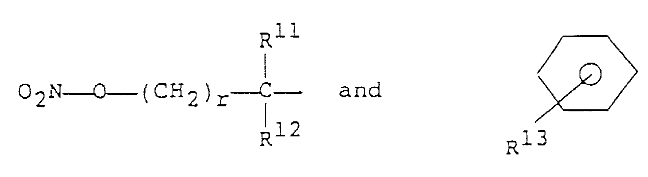 in which
R¹¹ denotes hydrogen, alkyl having 1 to 6 carbon atoms, substituted lower alkyl having 1 to 6 carbon atoms in which the substituent is halogen, hydroxyl, lower alkoxy having 1 to 6 carbon atoms, aryloxy, amino, lower alkylamino having 1 to 6 carbon atoms, acylamino, acyloxy, arylamino, mercapto, lower alkylthio having 1 to 6 carbon atoms, arylthio,
R¹² denotes hydrogen or lower alkyl having 1 to 6 carbon atoms,
R¹³ is nitratoalkyl having 1 to 6 carbon atoms,
r has the numerical values 0 to 10,
and in which
R¹, R^{1'} denote hydrogen or lower alkyl having 1 to 6 carbon atoms,
R², R^{2'} are hydrogen, lower alkyl having 1 to 6 carbon atoms, phenyl, methoxyphenyl, phenyl-lower-alkyl having 1 to 6 carbon atoms, methoxyphenyllower-alkyl having 1 to 6 carbon atoms, hydroxy-phenyl-lower-alkyl having 1 to 6 carbon atoms, hydroxylower-alkyl having 1 to 6 carbon atoms, alkoxy-lower-alkyl having 1 to 6 carbon atoms, amino-lower-alkyl having 1 to 6 carbon atoms, acylamino-lower-alkyl having 1 to 6 carbon atoms, mercapto-lower-alkyl having 1 to 6 carbon atoms or lower alkyl-thio-lower-alkyl having 1 to 6 carbon atoms,
R³, R^{3'} are amino, lower-alkylamino having 1 to 6 carbon atoms, di-loweralkylamino having 1 to 6 carbon atoms, aryl-lower-alkylamino having 1 to 6 carbon atoms, hydroxy-loweralkylamino having 1 to 6 carbon atoms, pyrrolidine, piperidine, morpholine, piperazine or amino-acid residues via peptide linkage,
R⁴ , R^{4'} are hydrogen or lower alkyl having 1 to 6 carbon atoms,
R⁵, R^{5'} denote like R⁴, R^{4'} hydrogen or lower alkyl,
R¹ and R², and R^{1'} and R^{2'}, can be linked together to form an alkylene bridge having 2 to 4 carbon atoms, an alkylene bridge having 2 to 3 carbon atoms and a sulphur atom, an alkylene bridge having 3 to 4 carbon atoms which contains a double bond or an alkylene bridge as above, substituted by hydroxyl, lower alkoxy having 1 to 6 carbon atoms, lower alkyl having 1 to 6 carbon atoms or di-lower-alkyl having 1 to 6 carbon atoms,
m, n, o, p, q and m', n', o', p' and q' have the numerical values 0 to 10,
which is characterized in that compounds of the general formula III 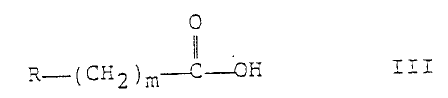 in which R denotes a group of the general formulae 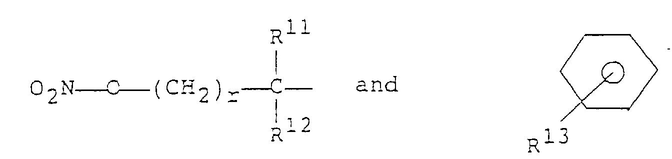 in which
R¹¹ denotes hydrogen, alkyl having 1 to 6 carbon atoms, substituted lower alkyl having 1 to 6 carbon atoms in which the substituent is halogen, hydroxyl, lower alkoxy having 1 to 6 carbon atoms, aryloxy, amino, lower alkylamino having 1 to 6 carbon atoms, acylamino, acyloxy, arylamino, mercapto, lower alkylthio having 1 to 6 carbon atoms, arylthio,
R¹² denotes hydrogen or lower alkyl having 1 to 6 carbon atoms,
R¹³ is nitratoalkyl having 1 to 6 carbon atoms,
m and r have the numerical values 0 to 10,
in the form of their free acids, reactive halides, azides, esters and anhydrides are condensed in a manner known per se with compounds of the general formula IV 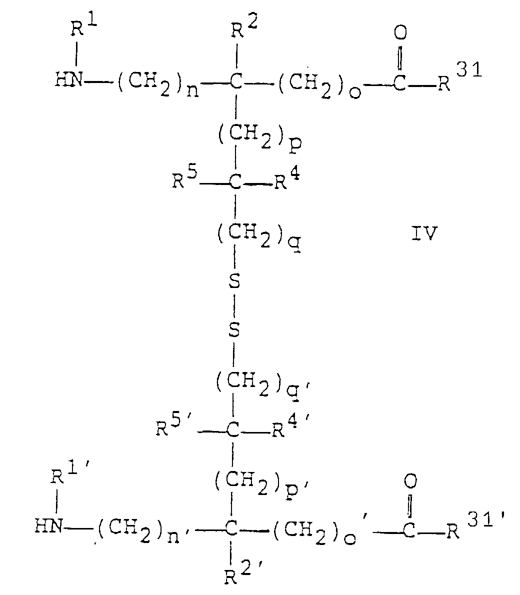 in which
R¹, R^{1'} denote hydrogen or lower alkyl having 1 to 6 carbon atoms,
R², R^{2'} are hydrogen, lower alkyl having 1 to 6 carbon atoms, phenyl, methoxyphenyl, phenyl-lower-alkyl having 1 to 6 carbon atoms, methoxyphenyllower-alkyl having 1 to 6 carbon atoms, hydroxy-phenyl-lower-alkyl having 1 to 6 carbon atoms, hydroxylower-alkyl having 1 to 6 carbon atoms, alkoxy-lower-alkyl having 1 to 6 carbon atoms, amino-lower-alkyl having 1 to 6 carbon atoms, acylamino-lower-alkyl having 1 to 6 carbon atoms, mercapto-lower-alkyl having 1 to 6 carbon atoms or lower alkyl-thio-lower-alkyl having 1 to 6 carbon atoms,
R³¹, R^{31'} denote hydroxyl or halogen,
R⁴ , R^{4'} are hydrogen or lower alkyl having 1 to 6 carbon atoms,
R⁵, R^{5'} denote like R⁴, R^{4'} hydrogen or lower alkyl having 1 to 6 carbon atoms,
R¹ and R², and R^{1'} and R^{2'}, can be linked together to form an alkylene bridge having 2 to 4 carbon atoms, an alkylene bridge having 2 to 3 carbon atoms and a sulphur atom, an alkylene bridge having 3 to 4 carbon atoms, which contains a double bond or an alkylene bridge as above, substituted by hydroxyl, lower alkoxy having 1 to 6 carbon atoms, lower alkyl having 1 to 6 carbon atoms or di-lower-alkyl having 1 to 6 carbon atoms,
m, n, o, p, q and m', n', o', p' and q' have the numerical values 0 to 10,
to give compounds of the general formula V 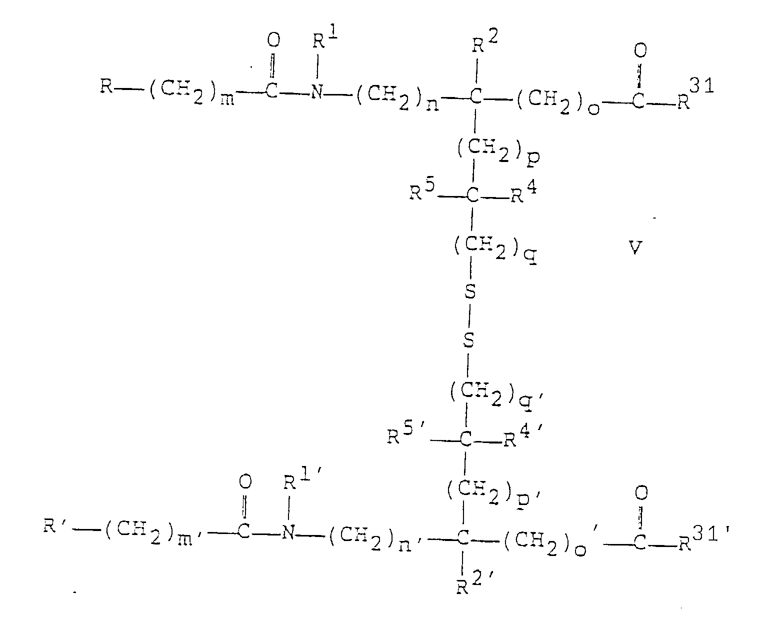 the latter are converted at their free or halogenated carboxyl functionalities with N-hydroxysuccinimide into activated carboxylic acid N-hydroxysuccinimide esters of the general formula VI 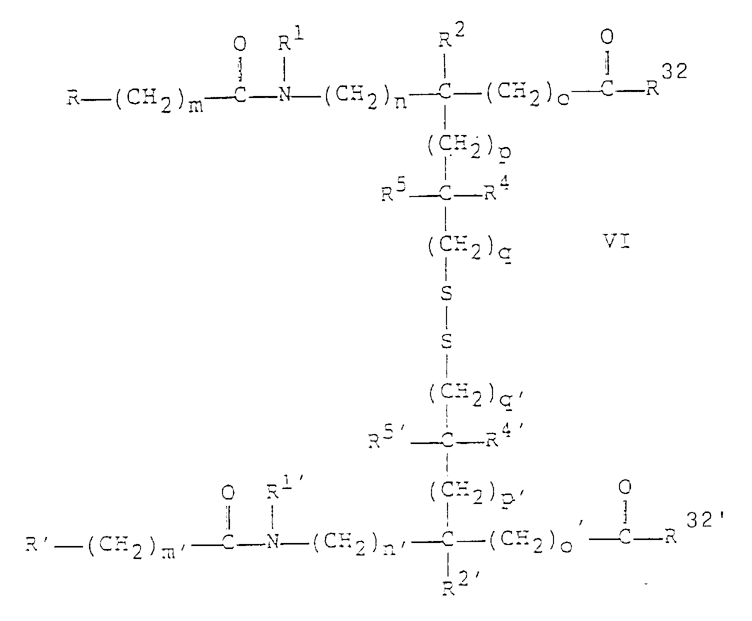 in which
R³² and R^{32'} denote, deviating from the abovementioned meaning, 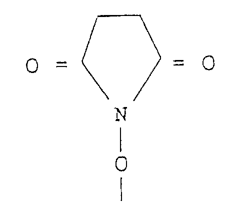 and the latter are subsequently reacted with ammonia, a primary or a secondary amine, in particular with lower alkylamine having 1 to 6 carbon atoms, di-lower-alkylamine having 1 to 6 carbon atoms, aryllower-alkylamine having 1 to 6 carbon atoms, hydroxylower-alkylamine having 1 to 6 carbon atoms, pyrrolidine, piperidine, morpholine, piperazine or amino acids to give the compounds of the general formula II.

10. Compounds which have the following names
- N-3-nitratopivaloyl-L-cysteinamide-glutathione mixed disulphide,
- N'-3-nitratopivaloyl-L-cysteine ethyl ester-glutathione mixed disulphide,
- N-3-nitratopivaloyl-L-cysteine ethyl ester-N'-acetyl-L-cysteine mixed disulphide,
- N-(3-nitratopivaloyl)-L-cysteine ethyl ester-D,L-penicillamine mixed disulphide,
- 2-acetylamino-3-[2-(2,2-dimethyl-3-nitrooxy-propionyl-amino)-2-ethoxycarbonylethyldisulphanyl]-3-methylbutyric acid.

11. Pharmaceutical containing one or more compound(s) according to Claims 1 to 16 and 10 as well as conventional excipients and ancillaries.

## Revendications

1. Composés selon la formule générale I : 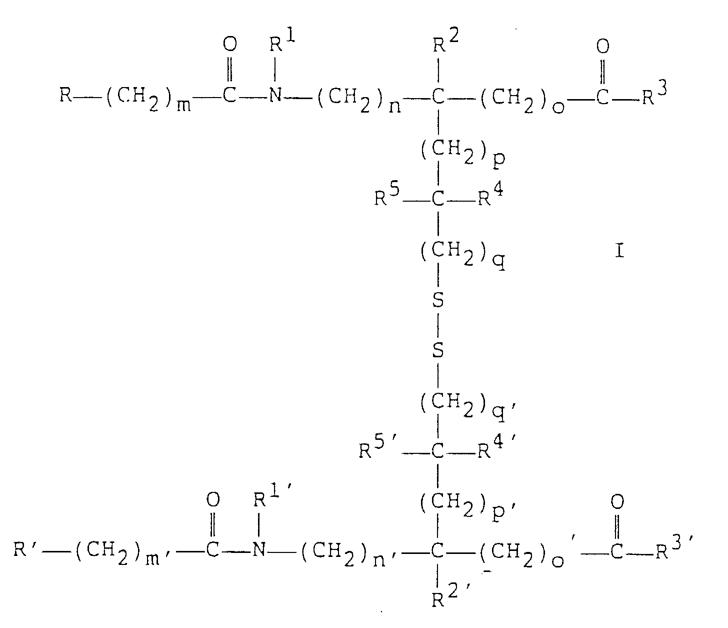 dans laquelle R et R' désignent des groupements de formule générale 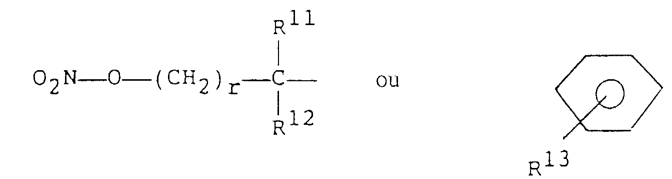 dans lesquels
R¹¹ est l'hydrogène, un alkyle possédant entre 1 et 6 atomes de carbone, un alkyle inférieur en C₁-C₆ substitué, dans lequel la substitution est effectuée avec un halogène, un groupement hydroxy, alcoxy inférieur en C₁-C₆, aryloxy, amino, alkylamino inférieur en C₁-C₆, acylamino, acyloxy, arylamino, mercapto, alkylthio inférieur en C₁-C₆. arylthio ;
R¹² est l'hydrogène ou un alkyle inférieur en C₁-C₆ ;
R¹³ est un nitroalkyle possédant entre 1 et 6 atomes de carbone ;
r a une valeur numérique de 0 à 10,
et dans lesquels
R¹, R^{1'} désignent l'hydrogène ou un alkyle inférieur en C₁-C₆ ;
R², R²' désignent l'hydrogène, un groupement alkyle inférieur en C₁-C₆, phényle, méthoxyphényle, phényl-alkyle inférieur en C₁-C₆, méthoxyphényl-alkyle inférieur en C₁-C₆, hydroxyphényl-alkyle inférieur en C₁-C₆, hydroxy-alkyle inférieur en C₁-C₆, alcoxy-alkyle inférieur en C₁-C₆, amino-alkyle inférieur en C₁-C₆, acylamino-alkyle inférieur en C₁-C₆, mercapto-alkyle inférieur en C₁-C₆ ou alkylthio inférieur en C₁-C₆-alkyle inférieur en C₁-C₆ ;
R³, R^{3'} désignent un groupement hydroxy, alcoxy inférieur en C₁-C_{6,} alokénoxy inférieur en C₁-C₆, dialkylamino inférieur en C₁-C₆-alcoxy inférieur en C₁-C₆; acylamino-alcoxy inférieur en C₁-C₆, acyloxy-alcoxy inférieur en C₁-C₆, aryloxy, aryl-alcoxy inférieur en C₁-C₆, aryloxy substitué ou aryl-alcoxy inférieur en C₁-C₆ substitué, où la substitution est effectuée par un méthyle, un halogène ou un groupement méthoxy ; amino, alkylamino inférieur en C₁-C₆; dialkylamino inférieur en C₁-C₆, arylalkylamino inférieur en C₁-C₆, hydroxyalkylamino inférieur en C₁-C₆, pyrrolidino, pipéridino, morpholino, de la pipérazino ou un ester d'acide aminé avec liaison peptidique ;
R⁴, R^{4'} désignent l'hydrogène ou un alkyle inférieur en C₁-C₆;
R⁵, R^{5'} désignent, comme R⁴, R^{4'}, l'hydrogène ou un alkyle inférieur en C₁-C₆;
R² et R³ ainsi que R^{2'} et R^{3'} peuvent être reliés entre eux par formation d'un ester ou d'un amide ;
R¹ et R² ainsi que R^{1'} et R^{2'} peuvent être reliés entre eux par formation d'un pont alkyle de 2 à 4 atomes de carbone, d'un pont alkyle de 2 à 3 atomes de carbone et un atome de soufre, d'un pont alkyle de 3 à 4 atomes de carbone comportant une double liaison ou un pont alkyle tel que décrit ci-dessus, substitué par un groupement hydroxy, alcoxy inférieur en C₁-C₆, alkyle inférieur en C₁-C₆ ou dialkyle inférieur en C₁-C₆ ;
m, n, o, p, q et m', n', o', p', q' possédant des valeurs numériques comprises entre 0 et 10,
ainsi que leurs sels physiologiquement compatibles.

2. Composés selon la Revendication 1, ***caractérisés en ce que*** leurs composants acide nitratoalcane-carboxylique ou nitratoalkylaryalcane-carboxylique ont une longueur de chaîne de 2 à 6 atomes de carbone, la chaîne pouvant être linéaire, ramifiée, racémique ou isomère optique.

3. Composés selon les Revendications 1 et 2, ***caractérisés en ce que*** les disulfures d'acides aminés qu'ils contiennent sont la cystine, l'homocystine ou le disulfure de pénicillamine.

4. Composés selon les Revendications 1 à 3, ***caractérisés en ce que*** les disulfures d'acides aminés sont présents sous la forme stéréochimique L.

5. Composés selon les Revendications 1 à 3, ***caractérisés en ce que*** les disulfures d'acides aminés sont présents sous la forme stéréochimique D,L.

6. Composés selon les Revendications 1 à 5, ***caractérisés en ce qu'***ils présentent les formules chimiques suivantes :
N,N'-di-(3-nitratopivaloyl)-L-cystine
N,N'-di-(3-nitratopivaloyl)-D,L-homocystine
diéthylester de la N,N'-di-(3-nitratopivaloyl)-L-cystine
diéthylester de la N,N'-di-(3-nitratopivaloyl)-D,L-homocystine
di-(2-méthyl-2-propyl)ester de la N,N'-di-(3-nitratopivaloyl)-L-cystine
diméthylester de la N,N'-di-(4-nitratométhylbenzoyl)-L-cystine
diméthylester de la N,N'-di-(3-nitratométhylbenzoyl)-L-cystine
N,N'-di-(4-nitratométhylbenzoyl)-L-cystine di-N,N'-butylamide
N,N'-di-(3-nitratométhylbenzoyl)-L-cystine di-N,N'-butylamide
N,N'-di-(4-nitratométhylbenzoyl)-L-cystine diamide
N,N' -di-(3-nitratométhylbenzoyl)-L-cystine diamide
disulfure de N,N'-di-(3-nitratopivaloyl)-L-pénicillamine diamide
N,N'-di-(3-nitratopivaloyl)-L-cystine diamide
N,N'-di-(3-nitratopivaloyl)-L-cystine di-N,N'-méthylamide
N,N'-di-(3-nitratopivaloyl)-L-cystine di-N,N'-butylamide
N,N'-di-(3-nitratopivaloyl)-L-cystine di-N,N'-tert-butylamide
N,N'-di-(3-nitratopivaloyl)-L-cystine dimorpholide
ester diisopropylique de la N,N'-di-(3-nitratopivaloyl)-L-cystine.

7. Procédé pour la production de composés selon les Revendications 1 à 6, ***caractérisé en ce que*** les composés sont convertis de manière connue. par condensation de l'acide nitratoalkylcarboxylique ou nitratoalkylarylalkylcarboxylique ou de leurs dérivés réactifs, avec les groupements amino d'un disulfure d'acide aminé.

8. Procédé pour la production de composés selon les Revendications 1 à 6, ***caractérisé en ce que*** les acides nitratoalkylcarboxylique ou nitratoalkylarylalkylcarboxylique correspondants ou leurs dérivés réactifs sont condensés avec les groupements amino d'acides aminés contenant des groupements thiol et les composés obtenus sont ensuite oxydés pour obtenir les disulfures correspondants par couplage.

9. Procédé pour la production de composés selon la formule générale II : 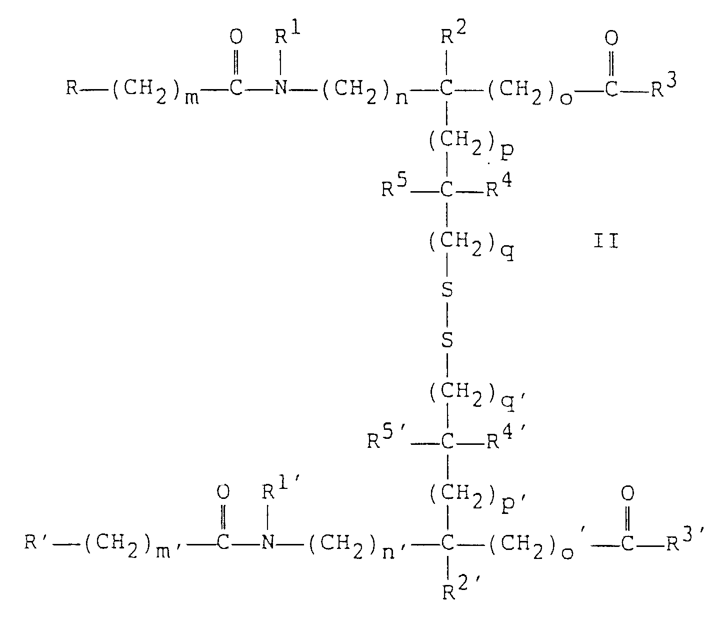 dans lesquels R et R' désignent des groupements de formule générale : 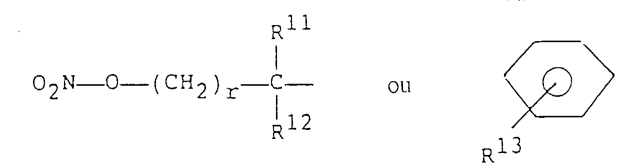 dans lesquels
R¹¹ est l'hydrogène, un alkyle possédant entre 1 et 6 atomes de carbone, un alkyle inférieur en C₁-C₆ substitué, dans lequel la substitution est effectuée par un halogène. un groupement hydroxy, alcoxy inférieur en C₁-C₆; aryloxy, amino, alkylamino inférieur en C₁-C₆, acylamino, acyloxy, arylamino, mercapto, C₁-C₆-alkylthio inférieur. arylthio ;
R¹² est l'hydrogène ou un alkyle inférieur en C₁-C₆ ;
R¹³ est un nitroalkyle possédant entre 1 et 6 atomes de carbone ;
r a une valeur numérique comprise entre 0 et 10,
et dans lesquels
R¹, R^{1'} désignent l'hydrogène ou un alkyle inférieur en C₁-C₆ ;
R², R^{2'} désignent l'hydrogène, un groupement alkyle inférieur en C₁-C₆, phényle. méthoxyphényle, phényl-alkyle inférieur en C₁-C₆, méthoxyphényl-alkyle inférieur en C₁-C₆, hydroxyphényl-alkyle inférieur en C₁-C₆, hydroxy-alkyle inférieur en C₁-C_{6,} alcoxy-alkyle inférieur en C₁-C₆, amino-alkyle inférieur en C₁-C₆, acylamino-alkyle inférieur en C₁-C₆, mercapto-alkyle inférieur en C₁-C₆ ou alkylthio inférieur en C₁-C₆-alkyle inférieur en C₁-C₆;
R³, R^{3'} désignent un groupement amino, aminoalkyle inférieur en C₁-C₆, dialkylamino inférieur en C₁-C₆, hydroxyalkylamino inférieur en C₁-C₆, pyrrolidino, pipéridino, morpholino, pipérazino ou un ester d'acide aminé avec liaison peptidique ;
R⁴, R^{4'} désignent l'hydrogène ou un alkyle inférieur en C₁-C₆ ;
R⁵, R^{5'} désignent, comme R⁴, R^{4'}, l'hydrogène ou un alkyle inférieur en C₁-C₆ ;
R¹ et R² ainsi que R^{1'} et R^{2'} peuvent être reliés entre eux par formation d'un pont alkyle de 2 à 4 atomes de carbone, d'un pont alkyle de 2 à 3 atomes de carbone et un atome de soufre. d'un pont alkyle de 3 à 4 atomes de carbone comportant une double liaison ou un pont alkyle tel que décrit ci-dessus, avec substitution par un groupement hydroxy, alcoxy inférieur en C₁-C₆, alkyle inférieur en C₁-C₆ ou dialkyle inférieur en C₁-C₆ ;
m, n, o, p, q et et m', n', o', p', q' possédant des valeurs numériques comprises entre 0 et 10,
***caractérisé en ce que*** des composés selon la formule générale III : 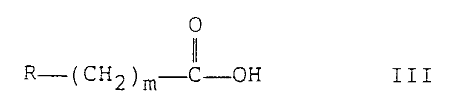 dans laquelle R désigne un groupe de formule générale : 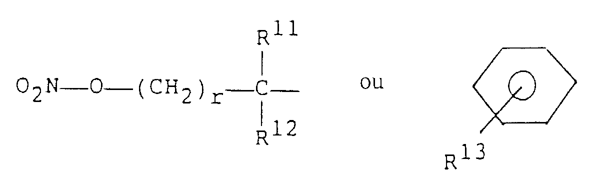 où
R¹¹ est l'hydrogène, un alkyle possédant entre 1 et 6 atomes de carbone, un alkyle inférieur en C₁-C₆ substitué, dans lequel la substitution est effectuée par un halogène, un groupement hydroxy, alcoxy inférieur en C₁-C₆, aryloxy, amino, alkylamino inférieur en C₁-C₆; acylamino, acyloxy, arylamino, mercapto, alkylthio inférieur en C₁-C₆, arylthio ;
R¹² est l'hydrogène ou un alkyle inférieur en C₁-C₆ ;
R¹³ est un nitroalkyle possédant entre 1 et 6 atomes de carbone ;
m a une valeur numérique comprise entre 0 et 10,
sont condensés sous la forme de leurs acides libres, d'halogénures d'acides réactifs, d'azotures d'acides, d'esters et d'anhydrides d'acides, de manière connue en soi, avec des composés selon la formule générale IV: 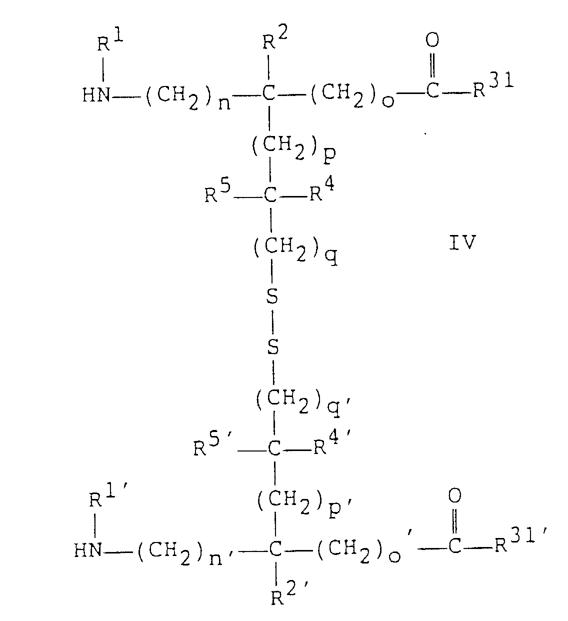 dans laquelle
R₁, R_{1'} désignent l'hydrogène ou un alkyle inférieur en C₁-C₆ ;
R², R^{2'} désignent l'hydrogène, un groupement alkyle inférieur en C₁-C₆, phényle, méthoxyphényle, phényl-alkyle inférieur en C₁-C₆, méthoxyphényl-alkyle inférieur en C₁-C₆. hydroxyphényl-alkyle inférieur en C₁-C₆; hydroxy-alkyle inférieur en C₁-C₆, alcoxy-alkyle inférieur en C₁-C₆, amino-alkyle inférieur en C₁-C₆, acylamino-alkyle inférieur en C₁-C₆ ou mercapto-alkyle inférieur en C₁-C₆ ;
R³¹, R^{31'} désignent un groupement hydroxy ou un halogène;
R⁴, R^{4'} désignent l'hydrogène ou un alkyle inférieur en C₁-C₆ ;
R⁵, R^{5'} désignent, comme R⁴, R^{4'}, l'hydrogène ou un alkyle inférieur en C₁-C₆ ;
R¹ et R² ainsi que R^{1'} et R^{2'} peuvent être reliés entre eux par formation d'un pont alkyle de 2 à 4 atomes de carbone, d'un pont alkyle de 2 à 3 atomes de carbone et un atome de soufre. d'un pont alkyle de 3 à 4 atomes de carbone comportant une double liaison ou un pont alkyle tel que décrit ci-dessus, avec substitution par un groupement hydroxy, alcoxy inférieur en C₁-C₆, alkyle inférieur en C₁-C₆ ou dialkyle inférieur en C₁-C₆ :
m, n, o, p, q et m', n', o', p', q' possédant des valeurs numériques comprises entre 0 et 10.
pour former des composés de formule générale V : 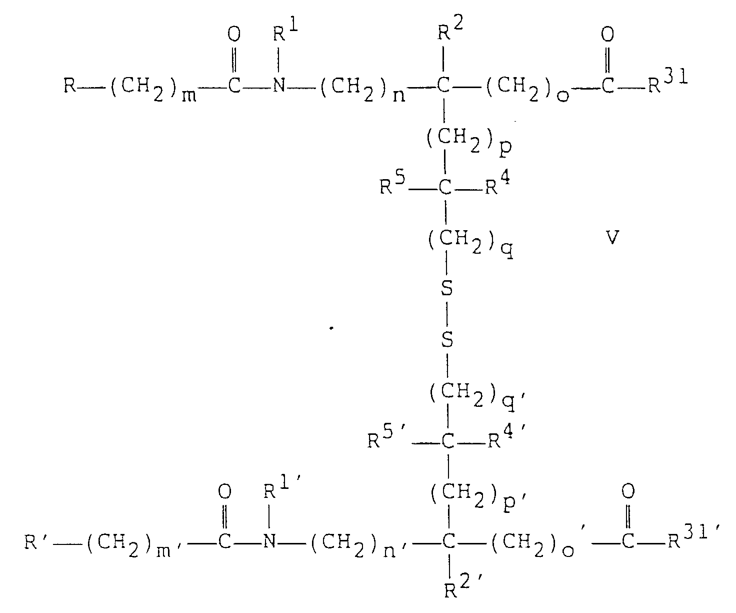 qui sont transformés, sur leurs fonctions acide carboxylique libres ou halogénées, à l'aide de N-hydroxysuccinimide, en esters N-hydroxysuccinimidiques d'acide carboxylique selon la formule générale VI: 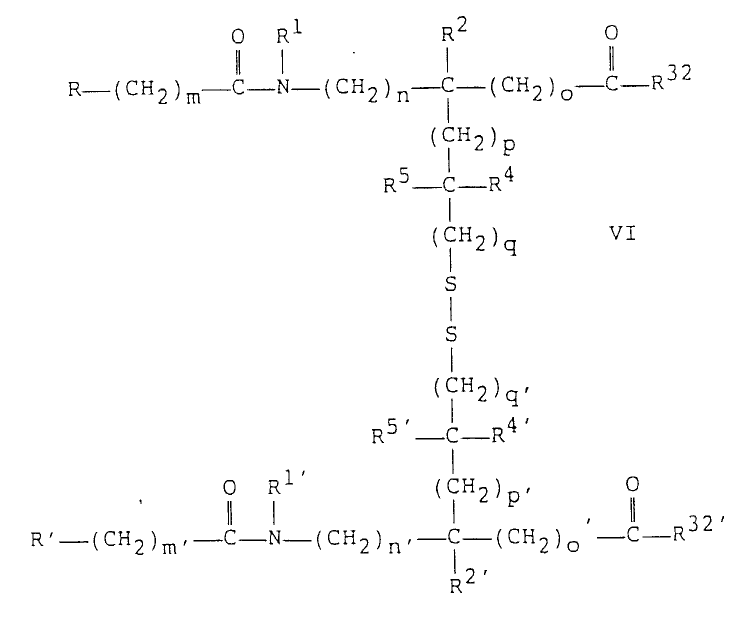 dans lesquels
R³² et R^{32'} désignent 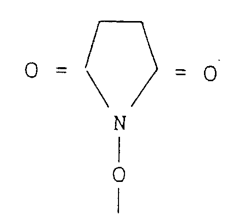
et sont ensuite convertis, avec de l'ammoniac, une amine primaire ou secondaire, notamment de une alkylamine inférieure en C₁-C₆, une dialkylamine inférieure en C₁-C₆, une hydroxyakylamine inférieure en C₁-C₆; la pyrrolidine, la pipéridine, la morpholine, la pipérazine ou des acides aminés, en composés selon la formule générale II.

10. Composés portant les dénominations suivantes :
- disulfure mixte de N'-3-nitratopivaloyl-L-cystéinamide et de glutathion
- disulfure mixte de N'-3-nitratopivaloyl-L-cystéinate d'éthyle et de glutathion
- disulfure mixte de N-3-nitratopivaloyl-L-cystéinate d'éthyle et de N'-acétyl-L-cystéine
- disulfure mixte de N-(3-nitratopivaloyl-L-cystéinate d'éthyle et de D,L-pénicillamine
- acide 2-acétylamino-3-[2-(2,2-diméthyl-3-nitrooxy-propionyl-amino)-2-éthoxycarbonyléthyldisulfanyl]-3-méthylbutyrique.

11. Médicaments contenant un ou plusieurs composés selon les Revendications 1 à 6 et 10 ainsi que des vecteurs et excipients usuels.
